(19) Europäisches Patentamt
European Patent Office
Office européen des brevets

(11) EP 0 782 568 B1

(12) **FASCICULE DE BREVET EUROPEEN**

(45) Date de publication et mention de la délivrance du brevet:
**24.04.2002 Bulletin 2002/17**

(21) Numéro de dépôt: **95932042.5**

(22) Date de dépôt: **22.09.1995**

(51) Int Cl.[7]: **C07D 209/16**, A61K 31/40, C07D 401/04, C07D 209/88, C07D 403/06

(86) Numéro de dépôt international:
**PCT/FR95/01220**

(87) Numéro de publication internationale:
**WO 96/09288 (28.03.1996 Gazette 1996/14)**

(54) **ETHERS AROMATIQUES DERIVES D'INDOLES COMME "5HT1-LIKE" LIGANDS**

VON INDOL ABGELEITETE AROMATISCHE ETHER ALS "5HT1-LIKE" LIGANDEN

AROMATIC ETHERS DERIVED FROM INDOLS SUCH AS 5HT1-LIKE LIGANDS

(84) Etats contractants désignés:
**AT BE CH DE DK ES FR GB GR IE IT LI LU MC NL PT SE**

(30) Priorité: **22.09.1994 FR 9411305**

(43) Date de publication de la demande:
**09.07.1997 Bulletin 1997/28**

(73) Titulaire: **PIERRE FABRE MEDICAMENT
92100 Boulogne (FR)**

(72) Inventeurs:
- **PEREZ, Michel
F-81100 Castres (FR)**
- **HALAZY, Serge
F-81090 Lagarrigue (FR)**
- **JOHN, Gareth, Win
F-81100 Les Salvages (FR)**
- **VALENTIN, Jean-Pierre
F-31320 Castanet-Tolosan (FR)**
- **PAUWELS, Peter
F-81440 Lautrec (FR)**

(74) Mandataire: **Warcoin, Jacques
Cabinet Régimbeau
20, rue de Chazelles
75847 Paris cedex 17 (FR)**

(56) Documents cités:
**WO-A-93/11106
WO-A-94/14770
WO-A-94/15916
WO-A-95/01334
WO-A-95/06638**

- **CHEMICAL ABSTRACTS, vol. 114, no. 19, 13 Mai 1991 Columbus, Ohio, US; abstract no. 178477w, GLENNON, RICHARD A. ET AL. '5-HT1D serotonin receptors: results of a structure-affinity investigation.' & DRUG DEV. RES., vol. 22, no. 1, pages 25-36,**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 782 568 B1

## Description

La présente invention se rapporte à de nouveaux éthers aromatiques dérivés d'indoles, ainsi qu'à leurs procédés de préparation, les compositions pharmaceutiques les contenant et leur utilisation comme médicament.

La sérotonine ou 5-hydroxytryptamine (5HT) joue un rôle important tant au niveau du système nerveux qu'au niveau cardio-vasculaire et des récepteurs sérotoninergiques ont été identifiés que ce soit au niveau central ou périphérique. Il est généralement admis que la sérotonine peut jouer un rôle important dans divers types de conditions pathologiques tels que certains désordres psychiatriques (anxiété, dépression, agressivité, attaques de panique, désordres compulsifs obsessionnels, schizophrénie, tendance au suicide), certains désordres neurodégénératifs (maladie d'Alzheimer, Parkinsonisme), la migraine, les céphalées et les troubles liés à l'alcoolisme (cf. E. Zifa et G. Fillion, Pharm. Reviews, 44, 401, 1992; A. Moulignier, Rev. Neuro. (Paris) 150, 3-15, 1994; S. Langer, N. Brunello, G. Racagni, J. Mendlecvicz, "Serotonin receptors subtypes : pharmacological significance and clinical implications" Karger ed.; 1992; B.E. Leonard, Int. Clin. Psychopharmacology, 7, 13-21, 1992; D.G. Grahame-Smith, Int. Clin. Psychopharmacology, 6, Suppl. 4, 6-13, 1992; E. Zifa, G. Fillion, Pharmacological Reviews, 44, 401-458, 1992; R.W. Fuller, J. Clin. Psychiatry, 53, 36-45, 1992).

Les composés selon la présente invention sont des composés nouveaux ayant une très haute affinité et une très bonne sélectivité pour les récepteurs communément appelés $5HT_{1\text{-like}}$ et plus particulièrement pour les récepteurs appelés $5HT_{1B}$ et $5HT_{1D}$, selon la nouvelle nomenclature récemment proposée par P. Humphrey, P. Hartig et D. Hoyer (TIPS, 14, 233-236, 1993).

Les médicaments incluant (seuls ou en association avec d'autres agents thérapeutiques), les principes actifs de la présente invention trouvent leur emploi dans le traitement tant curatif que préventif des maladies liées au dysfonctionnement des récepteurs $5HT_{1\text{-like}}$ incluant les récepteurs $5HT_{1B}$, $5HT_{1D\alpha}$ et $5HT_{1D\beta}$, à leur dérégulation ou à des modifications de l'activité du ligand endogène (généralement la sérotonine).

Les composés de la présente invention sont des ligands puissants et sélectifs des récepteurs $5HT_{1\text{-like}}$ qui peuvent agir comme agonistes, agonistes partiels ou antagonistes au niveau de ces récepteurs, et peuvent donc trouver une application dans les désordres liés à la sérotonine mentionnés ci-dessus.

La plupart des composés de la présente invention sont plus particulièrement des agonistes puissants (tant au niveau de leur affinité qu'au niveau de leur efficacité ou activité intrinsèque) et sélectifs des récepteurs $5HT_{1B}$ et $5HT_{1D}$. Les agonistes des récepteurs $5HT_{1\text{-like}}$ et plus particulièrement des récepteurs $5HT_{1D}$ présentent une activité vasoconstrictrice sélective et trouvent leur utilisation dans le traitement de la migraine et des désordres vasospastiques [(voir par exemple A. Doenicke et al., The Lancet, 1, 1309-1311, 1988; M.D. Ferrari, P.R. Saxena, Cephalalgia, 13, 151-165, 1993; S.J. Peroutka, Headache, 30, 5-11, 1990; M.A. Moskowitz, TiPS, 13, 307-311, 1992; W. Feniuk, P.P. Humphrey, M.S. Perren, H.E. Connor, E.T. Whalley, J. Neurol., 238, S57-S61, 1991; A.V. Deligonis, S.J. Peroutka, Headache, 31, 228-231, 1991)].

Les composés de la présente invention, qui sont, pour la plupart, des agonistes puissants et sélectifs des récepteurs $5HT_{1\text{-like}}$, trouvent donc plus particulièrement leur emploi dans le traitement curatif et prophylactique des crises de migraine "classique" (avec aura), "commune" (sans aura), l'algie vasculaire de la face, les céphalées chroniques vasculaires et des désordres vasospastiques.

L'état antérieur de la technique dans ce domaine est illustré notamment par :

- les demandes de brevet EP-0303507-A2, WO 93/14087, WO 94/02460, WO 92/14708 et les brevets US 4,839,377, GB 2124210A et GB 2162532A qui décrivent des sulfonamides dérivées de tryptamines (incluant le sumatriptan) comme antimigraineux.
- les demandes de brevet GB 2191488A, GB 2185020A et GB 2168347A qui décrivent des alkylamides dérivées de tryptamine.
- les demandes de brevets français FR 2 699 918 (30/12/92) et FR 2 707 639 (30/6/93) qui décrivent de nouveaux composés indoliques dérivés respectivement de pipérazines et d'arylamines comme ligands des récepteurs $5HT_{1B}$ - $5HT_{1D}$.
- la demande de brevet d'invention FR 2671971 qui décrit des dérivés 5-O-carboxyméthylés de la tryptamine qui ont une bonne affinité pour les récepteurs $5HT_{1D}$.
- les demandes de brevet européen 0313397, 0486666, 0494774-A1, 0494774, 0497512-A2, 0501568-A1, 0464558, 0548813-A1 et la demande de brevet WO 92/13856 et 93/11106 qui décrivent des dérivés hétérocycliques dérivés de tryptamine comme agonistes des récepteurs $5HT_{1\text{-like}}$.

La présente invention décrit une nouvelle classe d'éthers aromatiques dérivés d'indole qui se distingue de tous les dérivés les plus proches de l'art antérieur par leur structure chimique originale et différente, mais aussi, par leur profil biologique et leur potentiel thérapeutique puisque de nombreux composés selon la présente invention présentent une très forte affinité et sélectivité pour les récepteurs "$5HT_{1\text{-like}}$" et une efficacité agoniste remarquable. Ces

propriétés biologiques et pharmacologiques sont particulièrement mises en évidence lorsque de nombreux dérivés de la présente invention sont comparés au sumatriptan; les dérivés de la présente invention trouvent donc, pour la plupart, plus particulièrement leur utilité comme principes actifs de compositions médicamenteuses pour le traitement de la migraine et de divers troubles voisins.

La présente invention concerne des dérivés de formule générale (I)

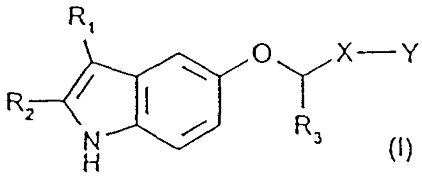

(I)

dans laquelle
R1 représente un reste aminé correspondant à l'une des formules (i) à (v) :

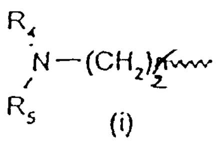

(i)

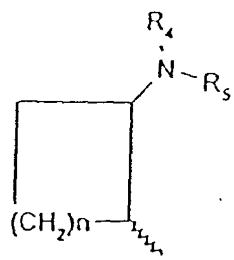

(ii)

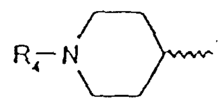

(iii)

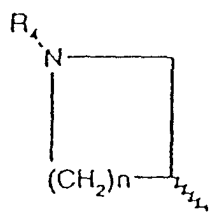

(iv)

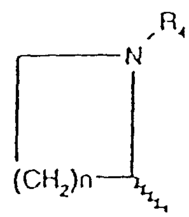

(v)

dans lesquelles n représente un nombre entier compris entre 1 et 5,
R4 représente un hydrogène, un groupement alkyle linéaire ou ramifié comprenant de 1 à 6 atomes de carbone ou un résidu du type $(CH_2)_mOR'$ dans lequel m représente un nombre entier compris entre 1 et 5, et R' un groupement alkyle linéaire ou ramifié comprenant de 1 à 6 atomes de carbone,
R5 représente un hydrogène, ou un groupement alkyle linéaire ou ramifié comprenant de 1 à 6 atomes de carbone,
$R_2$ représente un hydrogène, ou, $R_1$ et $R_2$, pris ensemble, forment un cycle à 6 atomes de carbone substitué par une fonction amine ($NR_4R_5$), à la condition que lorsque R1 représente un reste aminé de formule (i), $R_2$ représent un atome d'hydrogène,
$R_3$ représente un hydrogène, un résidu alkyle comprenant de 1 à 5 atomes de carbone ou un phényle.
X peut être omis ou représenter soit une chaîne alkyle linéaire ou ramifiée comprenant de 1 à 8 atomes de carbone soit un phényle pouvant être diversement substitué en diverses positions par un groupe alkyle linéaire ou ramifié comprenant de 1 à 6 atomes de carbone, un phényle un halogène, un alcool (OH), un nitrile (CN), un nitro ($NO_2$), un thiol (SH),
Y représente un résidu carbonylé ($COR_6$), sulfonylé ($SO_2R_6$), oxygéné ($OR_7$), aminé ($NHR_8$), nitrile (CN), nitro ($NO_2$), oxime (C = NOH) ou hydroxylamine (NHOH) dans lesquels $R_6$ représente $R'_6$, $OR'_6$ ou $NHR''_6$ où $R'_6$ et $R''_6$ représentent une chaîne alkyle linéaire ou ramifiée de 1 à 8 atomes de carbone, un cycloalkyle de 4 à 10 atomes de carbone, un résidu aromatique tel qu'un phényle, un benzyle, ou un phénétyle $R_7$ représente $R'_6$, $COR'_6$, $COOR'_6$ ou $CONHR'_6$ et

$R_8$ représente un hydrogène ou un résidu tel que $R''_6$, $COR'_6$, $CO_2R'_6$, $CONHR'_6$, $SO_2R'_6$ ou $SO_2NR'_6R''_6$ avec les restrictions suivantes :

- lorsque Y représente $COR_6$, lorsque X est omis et lorsque $R_3$ représente un hydrogène, alors $R_1$ doit être différent de $CH_2CH_2N(R_4R_5)$,
- lorsque $R''_6$ représente un reste aromatique, alors $R_1$ doit être différent de $CH_2CH_2N(R_4R_5)$, et
- Y est différent d'un groupe alkoxy,

leurs sels, hydrates, solvats acceptables pour l'usage thérapeutique.

Les composés de formule (I) contenant 1 ou plusieurs centres asymétriques présentent des formes isomères. Les racémiques et les énantiomères purs de ces composés font également partie de cette invention.

Parmi les sels acceptables pour l'usage thérapeutique des indoles de formule générale (I), on citera des sels formés par addition avec des acides organiques ou minéraux et par exemple les chlorhydrates, les bromhydrates, les sulfates, les fumarates et les maléates. D'autres sels peuvent être utiles dans la préparation des composés de formule (I), par exemple les adduits avec le sulfate de créatinine.

Les composés de la présente invention sont généralement préparés par condensation d'un dérivé indolique de formule générale (II).

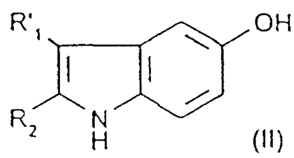

(II)

dans laquelle $R_2$ est défini comme précédemment et $R'_1$ peut être équivalent à $R_1$ ou à un précurseur de $R_1$ ( qui sera restauré en fin de synthèse par une réaction appropriée telle que par exemple la coupure d'un groupe protecteur) avec un dérivé de formule générale (III).

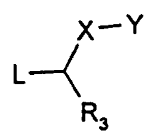

**(III)**

dans laquelle X, Y et $R_3$ sont définis comme précédemment et L représentent un groupe partant tel qu'un halogène (iode, brome ou chlore), un mésylate, un tosylate ou un triflate.

La préparation des dérivés de formule (I) par condensation des dérivés de formule (II) avec les dérivés de formule (III) peut être réalisée, d'une manière générale, en présence d'une base organique (NaH, KH, $Et_3N$, DBU, DBN, TMP, DIPEA, $^tBuOK$) ou inorganique ($K_2CO_3$, $KHCO_3$, $NaHCO_3$, $Cs_2CO_3$, KOH, NaOH, $CaCO_3$...) dans un solvant anhydre tel que le THF, la DMF, le DMSO, l'acétone, la diéthylcétone, la méthyléthylcétone, l'acétonitrile ou la DME à une température comprise entre 20° et 140°C, en présence ou non d'un sel comme catalyseur et qui peut être KI, $Bu_4NI$, LiI, $AgBF_4$, $AgClO_4$, $Ag_2CO_3$, KF, $Bu_4NF$ ou CsF. Le choix des conditions expérimentales et des réactifs pour réaliser la condensation entre les dérivés de formules (II) et (III) pour obtenir les dérivés de formule (I) est bien évidemment dépendant de la nature des substituants $R'_1$, $R_2$, X, Y, $R_3$ et L et sera réalisé selon les méthodes et techniques bien connues de l'homme de métier.

A titre d'exemple, quelques méthodes et variations sont décrites ci-dessous :

a. Dans le cas particulier des dérivés de formule (I) dans laquelle $R_2$ = H et $R_1$ représente $H_2N-CH_2-CH_2-$, une méthode appréciée de préparation consiste à condenser un dérivé de la sérotonine de formule (IIa)

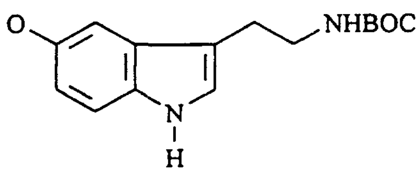

(IIa)

avec un électrophile de formule (III) selon les méthodes et techniques décrites précédemment suivie de la déprotection du N-t-butoxycarbonyle par réaction en milieu acide ($CF_3CO_2H$, HCl ou $H_2SO_4$).

b. Dans le cas particulier des dérivés de formule (Ib)

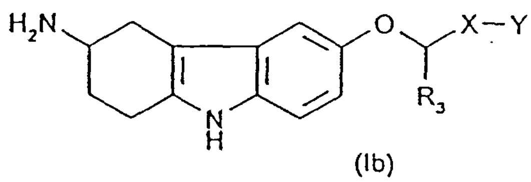

(Ib)

une méthode appréciée de préparation consiste à condenser un intermédiaire de formule (IIb)

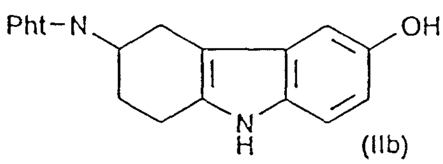

(IIb)

dans laquelle Pht représente un phtalimide avec un électrophile de formule (III) selon les méthodes et techniques décrites précédemment suivi de la déprotection du groupe phtalimide par réaction avec l'hydrazine ou l'éthylène diamine. L'intermédiaire de formule (IIb) (Cf. J. Chem. Soc., N°2, 325-326, 1970) est préparé par déméthylation sélective d'un intermédiaire de structure (IV) à l'aide de $BBr_3$.

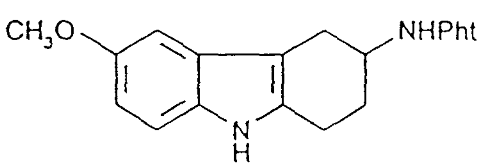

(IV)

c. Dans le cas particulier des composés de formule générale (Ic)

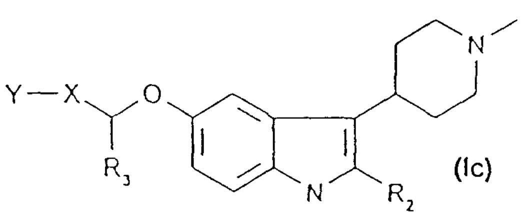

(Ic)

une méthode de préparation particulièrement appréciée consiste à condenser un intermédiaire de formule générale

(IIc)

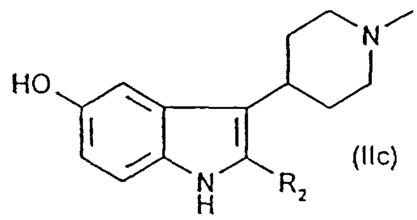

(IIc)

avec un électrophile de formule générale (III) selon les méthodes et techniques décrites précédemment. L'intermédiaire de formule (IIc) est préparé par condensation du 5-hydroxy-indole avec un dérivé carbonlé de formule (V)

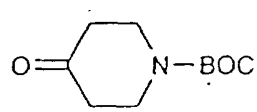

(V)

en présence de méthylate de sodium dans le méthanol ou d'hydroxyde de potassium dans un alcool suivi de la réduction de la double liaison par hydrogénation catalytique sur $PtO_2$ par l'hydrogène à pression atmosphérique dans le méthanol.

On comprendra que dans certaines réactions ou suites de réactions chimiques qui conduisent à la préparation de composés de formule générale (I) il soit nécessaire ou souhaitable de protéger des groupes ou fonctions sensibles dans les intermédiaires de synthèse afin d'éviter des réactions secondaires indésirables. Ceci peut être réalisé par l'utilisation de groupes protecteurs conventionnels tels que ceux décrits dans "Protective groups in organic synthesis", T.W. Greene, J. Wiley & Jones, 1981 et "Protecting groups" de P.J. Kocienski, Thieme Verlag, 1994. Les groupes protecteurs adéquats seront donc introduits puis enlevés au niveau des intermédiaires synthétiques les plus appropriés pour ce faire et en utilisant les méthodes et techniques décrites dans les références citées précédemment.

Doivent également être considérées comme faisant partie intégrale de la présente invention toutes les méthodes qui permettent de transformer un dérivé de formule (I) en un autre dérivé de formule I par les techniques et méthodes bien connues de l'homme de l'art. C'est ainsi, et à titre d'exemple, que les dérivés de formule (I) dans laquelle Y représente un nitrile (CN) peuvent être transformés en dérivés de formule (I) dans laquelle Y représente $CH_2NH_2$ par une réaction de réduction qui peut être effectuée par exemple à l'aide de Nickel de Raney.

De même, un dérivé de formule (I) dans laquelle Y représente un ester ($COOR'_6$) peut également être transformée en amide ($Y=CONH_2$) par réaction avec l'ammoniaque en présence de chlorure d'ammonium dans le méthanol. Les produits de formule (I) dans laquelle Y représente un ester ($CO_2R'_6$) peuvent être également transformés en alcool par réduction suivant les méthodes et techniques bien connues pour ce type de transformation telles que par exemple l'utilisation d'hydrure de lithium et d'aluminium dans un solvant tel que l'éther ou le THF. Les composés de formule générale (I) dans laquelle Y représente $NH_2$ sont des intermédiaires particulièrement appréciés pour la préparation de composés de formule (I), dans laquelle Y représente $NHRR''_6$, $NHCOR'_6$, $NHCO_2R'_6$, $NHCONHR'_6$, $NHSO_2R'_6$ ou $NHSO_2NR'_6R''_6$, par les méthodes et techniques bien connues pour transformer une amine primaire en amine secondaire, amide, carbamate, urée, sulfonamide ou sulfonurée.

Lorsque l'on désire isoler un composé selon l'invention à l'état de sel, par exemple de sel par addition avec un acide, on peut y parvenir en traitant la base libre de formule générale (I) par un acide approprié de préférence en quantité équivalente, ou par le sulfate de créatinine dans un solvant approprié.

Lorsque les procédés décrits ci-dessus pour préparer les composés de l'invention donnent des mélanges de stéréoisomères, ces isomères peuvent être séparés par des méthodes conventionnelles telles que la chromatographie préparative.

Lorsque les nouveaux composés de formule générale (I) possèdent un ou plusieurs centres asymétriques, ils peuvent être préparés sous forme de mélange racémique ou sous forme d'énantiomères que ce soit par synthèse énantionsélective ou par résolution. Les composés de formule (I) possédant au moins un centre asymétrique peuvent par exemple être séparés en leurs énantiomères par les techniques habituelles telles que la formation de paires diastéréomériques par formation d'un sel avec un acide optiquement actif tel que l'acide (-)-di-p-toluoyl-1-tartrique, l'acide (+)-di-p-toluoyl-1-tartrique, l'acide (+)-camphorsulfonique, l'acide (-)-camphorsulfonique , l'acide (+)-phénylpropionique, l'acide (-)-phénylpropionique, suivie par cristallisation fractionée et régénération de la base libre. les composés de formule (I) dans lesquels $R_1$ est un hydrogène comprenant au moins un centre asymétrique peuvent également être résolus par formation d'amides diastéréomériques qui sont séparés par chromatographie et hydrolysés pour libérer l'auxiliaire chiral.

La présente invention concerne également les sels, hydrates, solvats des composés exemplifiés ci-après.

Les exemples qui suivent illustrent l'invention sans toutefois en limiter la portée.

**Exemple 1 - Chlorhydrate du 4-[3-(2-amino-éthyl)-1H-indol-5-yloxyméthyl]-benzoate d'éthyle.**

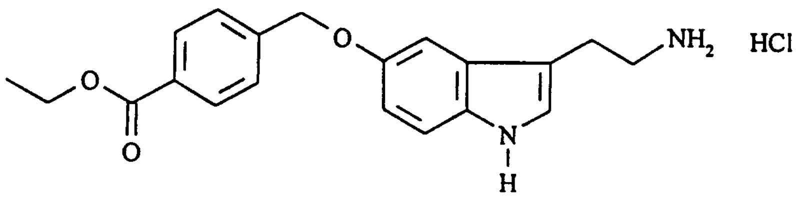

**1**

**1A -** *3-[2-N-(terbutoxycarbonyl)-amino-éthyl]-1H-indol-5-ol*

Le sel créatine sulfate monohydrate de la sérotonine (102 g, 252 mmol) est traité par le diterbutyle dicarbonate (82,6 g, 378 mmol) dans l'eau (2,1 l) en présence de soude 2N (420 ml) à température ambiante. Après 1 heure la réaction est diluée par de l'acétate d'éthyle (3 l) et agitée pendant 10 minutes. Les 2 phases formées sont séparées par décantation ; la phase organique est lavée à l'eau, séchée sur sulfate de sodium, filtrée puis évaporée à sec. Le sirop obtenu est chromatographié sur colonne de gel de silice éluée par un mélange dichlorométhane/méthanol (20: 1 ; v/v). Le composé pur est isolé sous forme de sirop marron (65,9 g ; 95 %).
Analyse élémentaire ($C_{15}H_{20}N_2O_3$), % calculés : C 65,20 ; H 7,30 ; N 10,14 ; % trouvés : C64.15 ; H 7.47 ; N 9.77.
RMN $^1H$, $CDCl_3$ (ppm) : 1,44 s, 9H; 2,86 t, 2H; 3,45 m, 2H; 4,68 s, 1H; 5,59 s, 1H; 6,77-7,26 m, 4H; 7,99 s, 1H.

**1B -** *4-[3-(2-N-(terbutoxycarbonyl)-amino-éthyl]-1H-indol-5-yloxyméthyl]-benzoate d'éthyle.*

Un mélange du 4-bromométhyl benzoate d'éthyle (950 mg; 3,9 mmol) et du composé 1A (600 mg; 2,17 mmol) dans la méthyléthylcétone (12 ml), en présence de carbonate de potassium (750 mg; 5,4 mmol) et d'iodure de potassium (144 mg; 0,87 mmol) est chauffé à reflux pendant une nuit. Le milieu est alors dilué au dichlorométhane, filtré sur célite, lavé à l'eau puis par une solution de chlorure de sodium. La phase organique est séchée sur sulfate de sodium, filtrée puis évaporée à sec.

Le sirop obtenu est chromatographié sur colonne de gel de silice élué par un mélange chloroforme/acétate d'éthyle (15:1; v/v). Le produit pur est obtenu sous forme de sirop incolore (517 mg; 54%).
RMN $^1H$, $CDCl_3$ (ppm) : 1,41 t, 3H; 1,50 s, 9H; 2,91 t, 2H; 3,44 t, 2H; 4,41 q, 2H; 5,18 s, 2H; 6,93 dd, 1H; 7,03-8,13 m, 8H.

***1**. Chlorhydrate du 4-[3-(2-amino-éthyl)-1H-indol-5-yloxyméthyl]-benzoate d'éthyle.*

Le produit 1B (370 mg; 0,843 mmol) en solution dans le toluène (10 ml) est traité par l'acide trifluoroacétique (1,5 ml). Après 3 h à température ambiante le milieu est dilué au dichlorométhane, lavé à la soude 2N puis à l'eau. La phase organique est séchée sur sulfate de sodium, filtrée et évaporée à sec. Le sirop obtenu est chromatographié sur colonne de gel de silice élué par un mélange dichlorométhane / méthanol / ammoniaque (90:9,5:0,5; v/v). Le produit pur est isolé sous forme de sirop incolore qui conduit, après traitement à l'acide chlorhydrique dans l'éther, au composé 1 (250 mg; 79%).
Analyse élémentaire ($C_{20}H_{23}N_2O_3Cl$), % calculés : C 64,08; H 6,18; N 7,47; % trouvés : C 64,04; H 6,21; N 6,91.
RMN $^1H$, DMSO-d6 (ppm) : 1,29 t, 3H, 2,98 m, 4H; 4,27 q, 2H; 5,19 s; 2H; 6,80 dd, 1H; 7,18 m, 3H; 7,58 d, 2H; 7,94 m, 5H; 10,82 s, 1H.
Point de fusion : 195-196°C

**Exemple 2 - Chlorhydrate du 4-[3-(2-amino-éthyl)-1H-indol-5-yloxyméthyl]-benzoate de méthyle.**

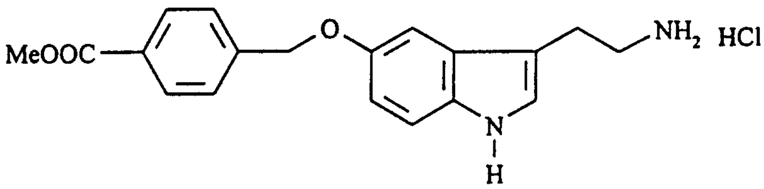

2

*2A- 4-[3-{2-N-(terbutoxycarbonyl)-amino-éthyl}-1H-indol-5-yloxyméthyl]-benzoate de méthyle.*

Le produit 2A est préparé à partir de 4-bromométhyle benzoate de méthyle (3,25 g; 14,18 mmol) et du composé 1A (2,17 g; 7,88 mmol) selon la procédure décrite pour la préparation du produit 1B.

Le sirop obtenu est chromatographié sur colonne de gel de silice élué par un mélange dichlorométhane/acétate d'éthyle (20:1 v/v). Le produit pur est isolé sous forme de sirop jaune pâle qui conduit, après traitement à l'acide chlorhydrique dans l'éther, au composé 1 (1,85 g; 55%).
RMN $^1$H, CDCl3 (ppm) : 1,43 s, 9H; 2,89 t, 2H; 3,40 t, 2H; 3,92 s, 3H; 5,17 s, 2H; 6,94 dd, 1H; 7,00-7,28 m, 3H; 7,53 d, 2H; 8,04 m, 3H.

**2** - *Chlorhydrate du 4-[3-(2-amino-éthyl)-1H-indol-5-yloxyméthyl]-benzoate de méthyle.*

Le produit 2 est obtenu à partir du composé 2A (600 mg; 1,41 mmol) selon la méthode décrite pour la préparation de l'exemple 1 à partir de 1B.

Le sirop obtenu est chromatographié sur colonne de gel de silice élué par un mélange dichlorométhane/méthanol/ammoniaque (90:9,5:0,5 v/v). Le produit pur est isolé sous la forme d'un solide beige qui conduit, après traitement à l'acide chlorhydrique dans l'éther, au composé 2 (367 mg; 71 %).
Analyse élémentaire ($C_{19}H_{21}N_2C_3Cl$), % calculés : C 63,24; H 5,87; N 7,76; % trouvés : C 64,29; H 6,00; N 7,69.
RMN$^1$H, DMSO-d6 (ppm) : 2,94 m, 4H; 3,86 s, 3H; 5,21 s, 2H; 6,81 dd, 1H; 7,19-8,01 m, 10H; 10,83 s, 1H.
Point de fusion : 230°C (décomposition).

**Exemple 3 - Chlorhydrate du {4-[3-(2-amino-éthyl)-1H-indol-5-yloxyméthyl]-phényl}-méthanol.**

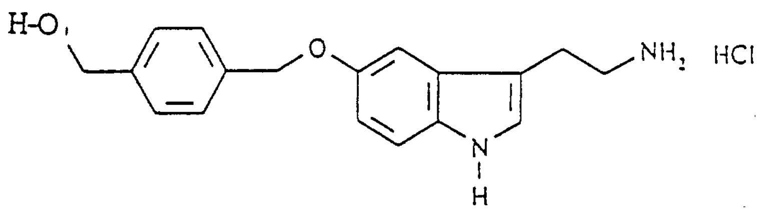

3

Le composé 2A (1,29 g; 3,03 mmol) en solution dans le tétrahydrofurane anhydre (30 ml) est traité, à 0°C et sous azote, par l'hydrure d'aluminium lithium (1M dans le THF) (3,64 ml; 3,64 mmol). Après 1h30 d'agitation à température ambiante le milieu est traité par un mélange sulfate de sodium/eau. Le précipité formé est filtré sur célite et le solvant est évaporé sous pression réduite. Le produit brut obtenu (1,2 g; 99%) est ensuite déprotégé selon la méthode décrite pour la préparation de l'exemple 1 à partir de 1B.

Le sirop obtenu est chromatographié sur colonne de gel de silice éluée par un mélange dichlorométhane/méthanol/ammoniaque (80:18,5:1,5; v/v). Le produit pur est isolé sous forme de sirop incolore qui conduit, après traitement à l'acide chlorhydrique dans l'éther, au composé 3 (466 mg; 46%).
Analyse élémentaire ($C_{18}H_{21}N_2O_2Cl$. 0,15 $H_2O$), % calculés : C 64,43; H 6,40; H 8,35; % trouvés : C 64,46; H 6,41;

N 8,06.
RMN $^1H$, DMSO-d6 (ppm) : 2,99 s, 4H; 4,99 s, 2H; 5,08 s, 2H; 6,78 dd, 1H; 7,19-7,45 m, 7H; 8,04 s, 3H; 10,83 s, 1H.
Point de fusion : 218°C

**Exemple 4 - Chlorhydrate du 4-[3-(2-amino-éthyl)-1H-indol-5-yloxyméthyl]-benzoate de propyle.**

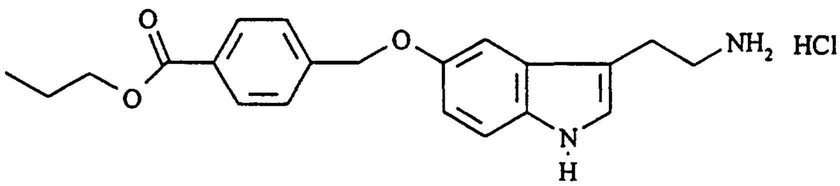

**4**

Un mélange du 4-bromométhyl-benzoate de propyle (1,6 g; 6,21 mmol) et du composé 1A (1,1 g; 4,14 mmol) dans la diméthylformamide (15ml) en présence de carbonate de césium (2,16 g; 6,62 mmol) est agité à température ambiante pendant une nuit. Le milieu est alors dilué à l'acétate d'éthyle, lavé à l'eau puis par une solution saturée de chlorure de sodium. La phase organique est séchée sur sulfate de sodium, filtrée et évaporée à sec.
Le sirop obtenu est chromatographié sur colonne de gel de silice éluée par un mélange dichlorométhane/ acétate d'éthyle (30:1; v/v). Le produit pur est isolé sous forme de sirop jaune pâle (570 mg; 30%). Ce produit est ensuite déprotégé selon les conditions décrites pour la préparation de l'exemple 1 à partir de 1B.
Le sirop obtenu est chromatographié sur colonne de gel de silice éluée par un mélange dichlorométhane/ méthanol/ammoniaque (85:14:1; v/v). Le produit pur est isolé sous forme de sirop incolore qui conduit, après traitement à l'acide chlorhydrique dans l'éther, au composé 4 (401 mg; 82%).
Analyse élémentaire ($C_{21}H_{25}N_2O_3Cl$), % calculés : C 64,86; H 6,48; N 7,20; Cl 9,12; % trouvés : C 64,56; H 6,42; N 7,17; Cl 9,53.
RMN $^1H$, DMSO-d6 (ppm) : 0,99 t, 3H; 1,67 m, 2H; 2,98 m, 2H; 4,22 t, 2H; 5,21 s, 2H; 6,82 dd, 1H; 7,21 m, 3H; 7,61 d, 2H; 7,96 m, 5H; 10,84 s, 1H.
Point de fusion : 205°C.

**Exemple 5 - Chlorhydrate du 2-[5-(4-nitro-benzyloxy)-1H-indol-3-yl]-éthylamine.**

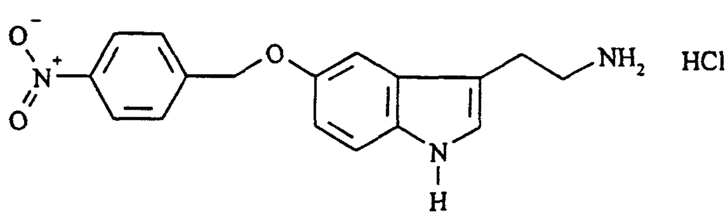

**5**

Le composé 5 est préparé à partir du chlorure de 4-nitrobenzyle (1 g; 5,8 mmol) et du composé 1A (895 mg, 3,24 mmol) selon la méthode décrite pour la préparation de l'exemple 1.
Le sirop obtenu est chromatographié sur colonne de gel de silice éluée par un mélange dichlorométhane/ méthanol/ammoniaque (80:19:1; v/v). Le produit pur est isolé sous forme de cristaux jaune-orangés qui conduit, après traitement à l'acide chlorhydrique dans l'éther, au composé 5 (659 mg, 59%).
Analyse élémentaire ($C_{17}H_{18}N_3O_3Cl$), % calculés : C 58,71; H 5,22; N 12,08; % trouvés : C 58,91; H 5,18; N 12,01.
RMN $^1H$, DMSO-d6 (ppm) : 3,00 m, 4H; 5,3 s, 2H; 6,83 dd, 1H; 7,24 m, 3H; 7,80 d, 2H; 8,06 s, 3H; 8,24 d, 2H; 10,87 s, 1H.
Point de fusion : 220°C (décomposition)

**Exemple 6 - Chlorhydrate du 2-[5-(4-méthyl-3-nitro-benzyloxy)-1H-indol-3-yl]-éthylamine.**

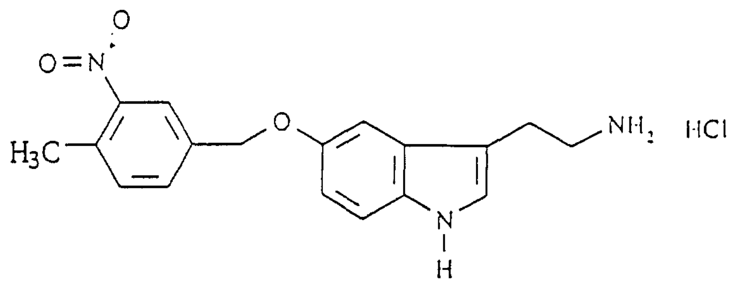

6

Le composé 6 est préparé à partir du chlorure de 4-méthyl-3-nitro-benzyle (1 g; 5,38 mmol) et du composé 1A (826 mg; 2,99 mmol) selon la méthode décrite pour la préparation de l'exemple 1.

Le sirop obtenu est chromatographié sur colonne de gel de silice éluée par un mélange dichlorométhane/méthanol/ammoniaque (80:19,5:0,5; v/v). Le produit pur est isolé sous forme de sirop jaune qui conduit, après traitement à l'acide chlorhydrique dans l'éther, au composé 6 (584 mg; 55%).
Analyse élémentaire ($C_{18}H_{20}N_3O_3Cl$), % calculés : C 59,75; H 5,57; N 11,61; % trouvés : C 60,21; H 5,58; N 11,53.
RMN $^1H$, DMSO-d6 (ppm) : 2,52 s, 3H; 3,00 m, 4H; 5,20 s, 2H; 6,83 dd, 1H; 7,20 m, 3H; 7,51 d, 1H; 7,72 d, 1H; 7,99 s, 3H; 8,10 d, 1H; 10,86 s, 1H.
Point de fusion :150°C.

**Exemple 7 - Chlorhydrate du 3-[3-(2-amino-éthyl)-1H-indol-5-yloxyméthyl]-benzoate de méthyle.**

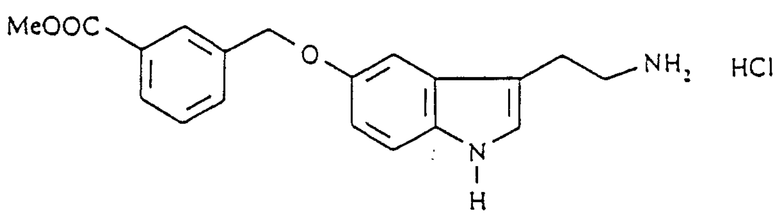

7

Le composé 7 est préparé à partir du 3-bromométhyle benzoate de méthyle (745 mg; 3,24 mmol) et du composé 1A (500 mg; 1,80 mmol) selon la méthode décrite pour la préparation de l'exemple 1.

Le sirop obtenu est chromatographié sur colonne de gel de silice éluée par un mélange dichlorométhane/méthanol/ammoniaque (90:9,5:0,5; v/v). Le produit pur est isolé sous forme de sirop incolore qui conduit, après traitement à l'acide chlorhydrique dans l'éther, au composé 7 (383 mg, 59%).
Analyse élémentaire ($C_{19}H_{21}N_2O_3Cl$), % calculés : C 63,24; H 5,87; N 7,76; % trouvés : C 63,15; H 5,82; N 7,74.
RMN $^1H$, DMSO-d6 (ppm) : 3,01 s, 4H; 3,86 s, 3H; 5,20 s, 3H; 6,82 dd, 1H; 7,20 m, 3H; 7,55 m, 1H; 7,75-8,12 m, 6H; 10,87 d, 1H.
Point de fusion : 185-186°C.

**Exemple 8 - Chlorhydrate du 2-[5-(2-nitro-benzyloxy)-1H-indol-3-yl]-éthylamine.**

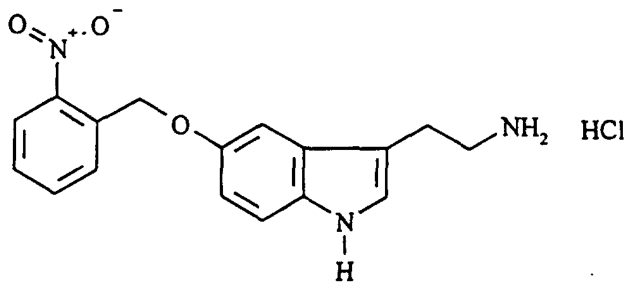

**8**

Le composé 8 est préparé à partir du bromure de 2-nitro-benzyle (703 mg; 3,24 mmol) et du composé 1A (600 mg; 2,16 mmol) selon la méthode décrite pour la préparation de l'exemple 1.

Le sirop obtenu est chromatographié sur colonne de gel de silice éluée par un mélange dichlorométhane/méthanol/ammoniaque (90:9,5:0,5; v/v). Le produit pur est isolé sous forme de solide orange qui conduit, après traitement à l'acide chlorhydrique dans l'éther, au composé 8 (439 mg; 58%).

Analyse élémentaire ($C_{17}H_{18}N_3O_3Cl$), % calculés : C 57,81; H 5,31; N 11,90; % trouvés : C 57,73; H 5,15; N 11,65.

RMN $^1H$, DMSO-d6 (ppm) : 3,00 s, 4H; 5,44 s, 2H; 6,79 dd, 1H; 7,18-7,30 m, 3H; 7,57-8,11 m, 7H; 10,91 d, 1H.

Point de fusion : 238°C (décomposition)

**Exemple 9 - Chlorhydrate du 2-[3-(2-amino-éthyl)-1H-indol-5-yloxyméthyl]-benzoate d'éthyle.**

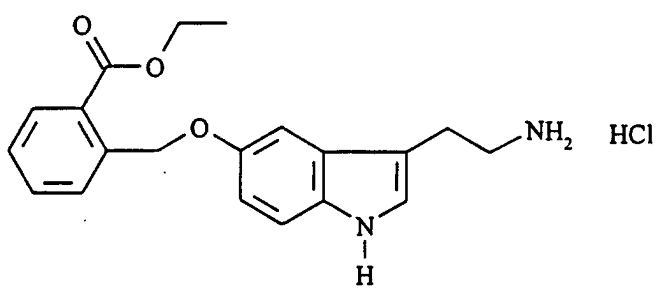

**9**

Le composé 2 est préparé à partir du 2-bromométhyle benzoate d'éthyle (4,1g; 16,84 mmol) et du composé 1A (2,6 g; 9,36 mmol) selon la méthode décrite pour la préparation de l'exemple 1.

Le sirop obtenu est chromatographié sur colonne de gel de silice éluée par un mélange dichlorométhane/méthanol/ammoniaque (90:9,5:0,5; v/v). Le produit pur est isolé sous forme de sirop incolore qui conduit, après traitement à l'acide chlorhydrique dans l'éther, au composé 9 (1,84 g; 52%).

Analyse élémentaire ($C_{20}H_{23}N_2O_3Cl$), % calculés : C 64,08; H 6,18; N 7,47; % trouvés : C 63,82; H 6,25; N 7,22.

RMN $^1H$, DMSO-d6 (ppm) : 1,20 t, 3H; 2,97 s, 4H; 4,20 q, 2H; 5,38 s, 2H; 6,74 dd, 1H; 7,12-8,04 m, 10H; 10,86 s, 1H.

Point de fusion : 235°C (décomposition)

**Exemple 10 - Chlorhydrate du 2-[3-(2-amino-éthyl)-1H-indol-5-yloxyméthyl]-1-phényl-éthanone**

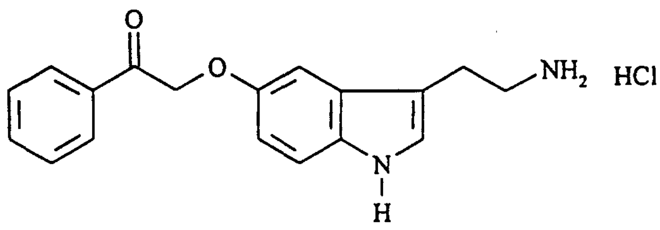

**10**

Le composé 10 est préparé à partir du 2-chloro-1-phényl-éthanone (1 g; 6,46 mmol) et du composé 1A (993 mg; 3,59 mmol) selon la méthode décrite pour la préparation de l'exemple 1.

Le sirop obtenu est chromatographié sur colonne de gel de silice éluée par un mélange dichlorométhane/méthanol/ammoniaque (80:19:1; v/v). Le produit pur est isolé sous forme de sirop jaune qui conduit, après traitement à l'acide chlorhydrique dans l'éther, au composé 10 (254 mg; 22%).

Analyse élémentaire ($C_{18}H_{19}N_2O_2Cl$), % calculés : C 65,35; H 5,79; N 8,47; % trouvés : C 64,41. H 5,75; N 8,60

RMN $^1$H, DMSO-d6 (ppm) : 2,98 m, 4H; 5,52 s, 2H; 6,80 dd, 1H; 7,20 m, 3H; 7,52-7,68 m, 3H; 8,05 m, 5H; 10,87 s, 1H.

Point de fusion : 131 °C

**Exemple 11 - Chlorhydrate du 2-[3-(2-amino-éthyl)-1H-indol-5-yloxy]-1-(4-méthoxy-phényl)-éthanone.**

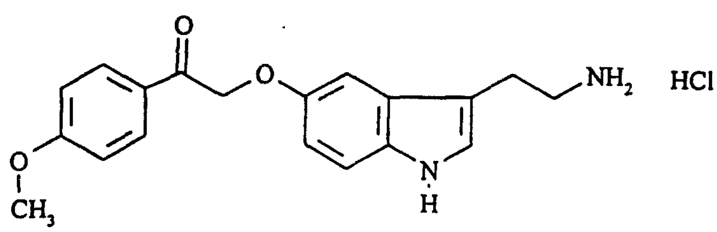

**11**

Le composé 11 est préparé à partir du bromure de 4-méthoxy-phénacyle (622 mg; 2,7 mmol) et du composé 1A (500 mg; 1,81 mmol) selon la méthode décrite pour la préparation de l'exemple 4.

Le sirop obtenu est chromatographié sur colonne de gel de silice éluée par un mélange dichlorométhane/méthanol/ammoniaque (80:18,5:1,5; v/v). Le produit pur est isolé sous forme de sirop jaune qui conduit, après traitement à l'acide chlorhydrique dans l'éther, au composé 11 (256 mg; 40%).

Analyse élémentaire ($C_{19}H_{21}N_2O_3Cl$, $H_2O$), % calculés : C 60,24; H 6,12; N 7,39; Cl 9,36; % trouvés : C 60,29; H 5,95; N 7,28; Cl 9,29.

RMN $^1$H, DMSO-d6 (ppm) : 3,01 m, 4H; 3,85 s, 3H; 5,42 s, 2H; 6,78 dd, 1H; 7,05-7,27 m, 5H; 7,93-8,05 m, 5H; 10,83 s, 1H.

Point de fusion :144°C

**Exemple 12 - Chlorhydrate du 4-[3-(2-amino-éthyl)-1H-indol-5-yloxy)-butyrate d'éthyle**

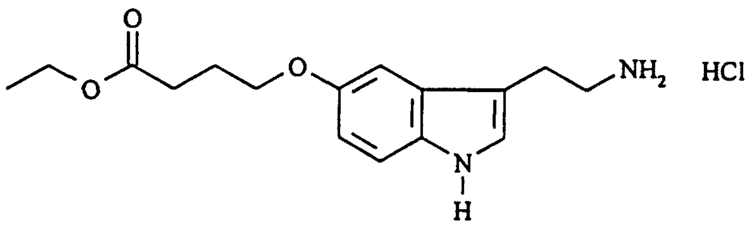

**12**

Le composé 12 est préparé à partir du 4-bromobutyrate d'éthyle (1,87 ml; 13,03 mmol) et du composé 1A (2 g; 7,24 mmol) selon la méthode décrite pour la préparation de l'exemple 1.

Le sirop obtenu est chromatographié sur colonne de gel de silice éluée par un mélange dichlorométhane/méthanol/ammoniaque (80:18,5:1,5; v/v). Le produit pur est isolé sous forme de sirop incolore qui conduit, après traitement à l'acide chlorhydrique dans l'éther, au composé 12 (1,14 g; 48%).
Analyse élémentaire ($C_{16}H_{23}N_2O_3Cl$), % calculés : C 58,80; H 7,09; N 8,57; % trouvés : C 58,92; H 6,93; N 8,58.
RMN $^1$H, DMSO-d6 (ppm) : 1,18 t, 3H; 1,94 m, 2H; 2,50 t, 2H; 2,99 s, 4H; 4,01 m, 4H;, 6,71 dd, 1H; 7,06-7,26 m, 3H; 8,07 s, 1H; 10,82 s, 1H.
Point de fusion : 173°C (décomposition)

**Exemple 13 - Chlorhydrate du 5-[3-(2-amino-éthyl)-1H-indol-5-yloxy]-pentanoate d'éthyle.**

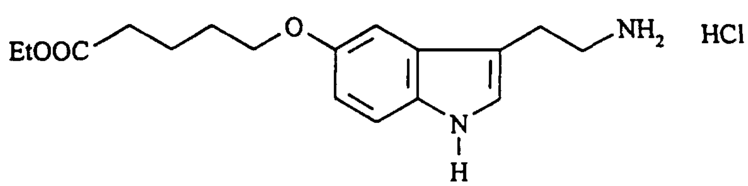

**13**

Le composé 13 est préparé à partir du 5-bromovalérate d'éthyle (3,1 ml; 19,54 mmol) et du composé 1A (3 g; 10,86 mmol) selon la méthode décrite pour la préparation de l'exemple 1.

Le sirop obtenu est chromatographié sur colonne de gel de silice éluée par un mélange dichlorométhane/méthanol/ammoniaque (80:19:1; v/v). Le produit pur est isolé sous forme de sirop beige qui conduit, après traitement à l'acide chlorhydrique dans l'éther, au composé 13 (1,87 g; 51%).
Analyse élémentaire ($C_{17}H_{25}N_2O_3Cl$), % calculés : C 59,91; H 7,39; N 8,22; % trouvés : C 59,51; H 7,27; N 8,01.
RMN $^1$H, DMSO-d6 (ppm) : 1,16 t, 3H; 1,69 m, 4H;, 2,32 t, 2H; 2,97 m, 4H; 3,98 m, 4H; 6,69 dd, 1H; 7,05-7,24 m, 3H; 8,05 s, 3H; 10,81 s, 1H.
Point de fusion : 177°C

**Exemple 14 - Chlorhydrate du 6-[3-(2-amino-éthyl)-1H-indol-5-yloxy]-hexanoate d'éthyle.**

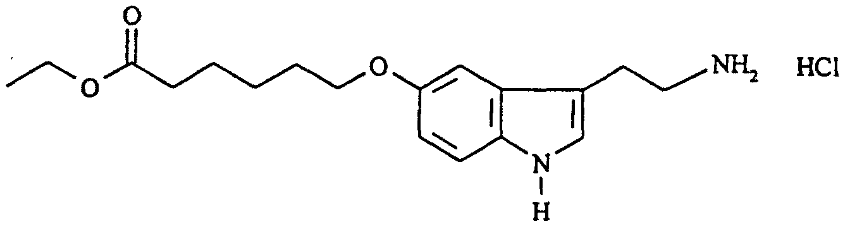

**14**

Le composé 14 est préparé à partir du 6-bromohexanoate d'éthyle (1,3 ml, 7,24 mmol) et du composé 1A (1 g; 3,62 mmol) selon la méthode décrite pour la préparation de l'exemple 1.

Le sirop obtenu est chromatographié sur colonne de gel de silice éluée par un mélange dichlorométhane/méthanol/ammoniaque (80:19:1; v/v). Le produit pur est isolé sous forme de sirop jaune pâle qui conduit, après traitement à l'acide chlorhydrique dans l'éther, au composé 14 (746 mg; 58%).
Analyse élémentaire ($C_{18}H_{27}N_2O_3Cl$), % calculés : C 60,92; H 7,67; N 7,89; Cl 9,99; % trouvés : C 60,72; H 7,64; N 7,80; Cl 10,03.
RMN $^1H$, DMSO-d6 (ppm) : 1,16 t, 3H; 1,38-1,78 m, 6H; 2,30 t, 2H; 2,98 s, 4H; 3,91-4,09 m, 4H; 6,69 dd, 1H; 7,05-7,24 m, 3H; 8,10 s, 3H; 10,81 s, 1H.
Point de fusion : 170°C.

**Exemple 15 - Chlorhydrate du 6-[3-(2-amino-éthyl)-1H-indol-5-yloxy]-hexanoate de benzyle.**

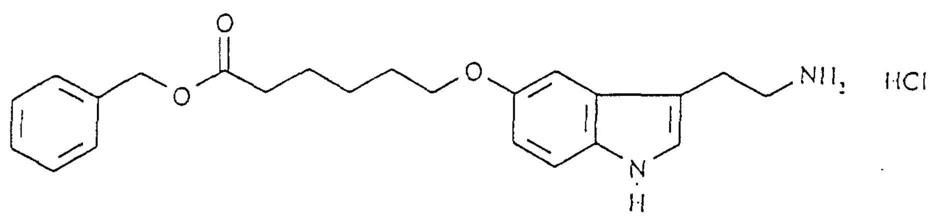

15

Le composé 15 est préparé à partir du 6-bromohexanoate de benzyle (929 mg; 3,25 mmol) et du composé 1A (500 mg; 1,81 mmol) selon la méthode décrite pour la préparation de l'exemple 1.

Le sirop obtenu est chromatographié sur colonne de gel de silice éluée par un mélange dichlorométhane/méthanol/ammoniaque (85:14:1; v/v). Le produit pur est isolé sous forme de sirop jaune qui conduit, après traitement à l'acide chlorhydrique dans l'éther, au composé 15 (479 mg; 64%).
Analyse élémentaire ($C_{23}H_{29}N_2O_3Cl$), % calculés : C 66,26; H 7,01; N 6,72; Cl 8,50; % trouvés : C 66,05; H 6,95; N 6,65; Cl 8,40.
RMN $^1H$, DMSO-d6 (ppm) : 1,44-1,76 m, 6H; 2,40 t, 2H; 2,99 s, 4H; 3,95 t, 2H; 5,09 s, 2H; 6,70 dd, 1H; 7,06-7,36 m, 8H; 8,05 s, 3H; 10,82 s, 1H.
Point de fusion : 130°C.

**Exemple 16 - Chlorhydrate du 6-[3-(2-amino-éthyl)-1H-indol-5-yloxy]-hexanoate d'isopropyle.**

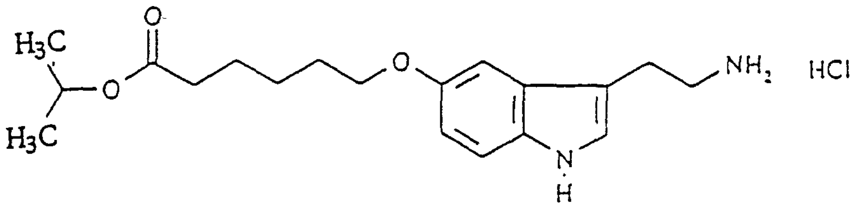

16

Le composé 16 est préparé à partir du 6-bromohexanoate d'isopropyle (772 mg; 3,25 mmol) et du composé 1A (500 mg; 1,81 mmol) selon la méthode décrite pour la préparation de l'exemple 1.

Le sirop obtenu est chromatographié sur colonne de gel de silice éluée par un mélange dichlorométhane/méthanol/ammoniaque (80:19:1; v/v). Le produit pur est isolé sous forme de sirop incolore qui conduit, après traitement à l'acide chlorhydrique dans l'éther, au composé 16 (369 mg; 55%).

Analyse élémentaire ($C_{19}H_{29}N_2O_3Cl$), % calculés : C 61,86; H 7,92; N 7,59; Cl 9,61; % trouvés : C 61,91; H 7,93; N 7,49; Cl 9,64.

RMN $^1H$, DMSO-d6 (ppm) : 1,26 d, 6H; 1,43-1,76 m, 6H; 2,28 t, 2H; 2,98 m, 4H; 3,95 t, 2H; 4,88 m, 1H; 6,70 dd, 1H; 7,05-7,26 m, 3H; 8,01 s; 3H; 10,80 s, 1H.

Point de fusion : 137°C

**Exemple 17 - Chlorhydrate du 6-[3-(2-amino-éthyl)-1H-indol-5-yloxy]-hexanitrile**

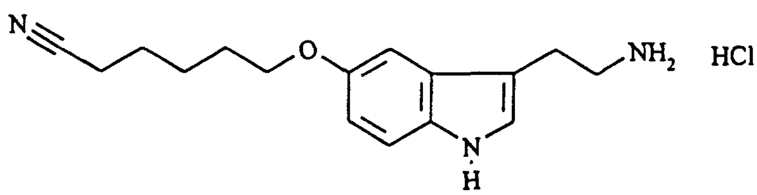

**17**

*17A - 6-[3-(2-N-(terbutoxycarbonyl)-amino-éthyl)-1H-indol-5-yloxy]-hexanitrile*

Le composé 17A est préparé à partir du 6-bromocapronitrile (2,87 g, 16,29 mmol) et du composé 1A (2,5 g; 9,04 mmol) selon la méthode décrite pour la préparation de l'exemple 1B.

Le sirop obtenu est chromatographié sur colonne de gel de silice éluée par un mélange dichlorométhane/acétone (20:1; v/v). Le produit pur est isolé sous forme de sirop jaune (3,17 g; 94%).

***17 -** Chlorhydrate du 6-[3-(2-amino-éthyl)-1H-indol-5-yloxy]-hexanitrile*

Le composé 17A (650 mg; 1,75 mmol) est ensuite déprotégé selon la méthode décrite pour la préparation de l'exemple 1 à partir de 1B.

Le sirop obtenu est chromatographié sur colonne de gel de silice éluée par un mélange dichlorométhane/méthanol/ammoniaque (80:19:1; v/v). Le produit pur est isolé sous forme de sirop jaune pâle qui conduit, après traitement à l'acide chlorhydrique dans l'éther, au composé 17 (436 mg; 81%).

Analyse élémentaire ($C_{16}H_{22}N_3OCl$, 0,5 $H_2O$), % calculés : C 60,65; H 7,32; N 13,29; Cl 11,18; % trouvés : C 60,67; H 7,03; N 12,92; Cl 11,54.

RMN $^1H$, DMSO-d6 (ppm) : 1,56-1,73 m, 6H; 2,48 t, 2H; 2,96 s, 4H; 3,95 t, 2H; 6,70 dd, 1H; 7,05-7,24 m, 3H; 8,02 s, 3H; 10,80 s, 1H.

Point de fusion : 124°C.

**Exemple 18 - Chlorhydrate du 6-[3-(2-amino-éthyl)-1H-indol-5-yloxy]-hexylamine.**

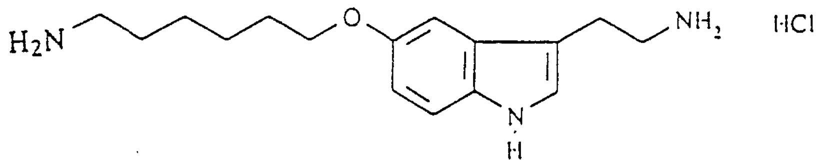

18

*18A - 6-{3-[2-N-(terbutoxycarbonyl)-amino-éthyl]-1H-indol-5-yloxy}-hexylamine.*

Le composé 17A (2,49 g; 6,69 mmol) en solution dans le tétrahydrofurane (93 ml), en présence d'ammoniaque (6,2 ml) et de Nickel de Raney (2 spatules), est soumis à une pression atmosphérique d'hydrogène pendant 25 heures. Le mélange est ensuite filtré sur célite et le solvant évaporé à sec.

Le sirop obtenu est chromatographié sur colonne de gel de silice éluée par un mélange dichlorométhane/méthanol/ammoniaque (80:19:1; v/v). Le produit pur est isolé sous forme de sirop incolore (2,45 g; 97%).

RMN $^1$H, DMSO-d6 (ppm) : 1,37 m, 15H; 1,68 m, 2H; 2,49 m, 2H; 2,73 t, 2H; 3,14 m, 2H; 3,93 t, 2H; 6,67 dd, 1H; 6,87-7,24 m, 4H; 10,61 s, 1H.

*18 - Chlorhydrate du 6-[3-(2-amino-éthyl)-1H-indol-5-yloxy]-hexylamine.*

Le produit 18A (800 mg; 2,13 mmol) est ensuite déprotégé selon la méthode décrite pour la préparation de l'exemple 1 à partir de 1B.

Le sirop obtenu est chromatographié sur colonne de gel de silice éluée par un mélange dichlorométhane/méthanol/ammoniaque (75:20:5; v/v). Le produit pur est isolé sous forme de sirop incolore qui conduit, après traitement à l'acide chlorhydriqué dans l'éther, au composé 18 (556 mg; 75%).

Analyse élémentaire ($C_{16}H_{27}N_3OCl_2$, $H_2O$), % calculés : C 52,46; H 7,98; N 11,47; Cl 19,36; % trouvés : C 52,41; H 7,46; N 11,05; Cl 21,75.

RMN $^1$H, DMSO-d6 (ppm) : 1,45-1,74 m, 8H; 2,76 m, 2H; 3,00 s, 4H; 3,99 t, 2H; 6,72 dd, 1H; 7,10-7,27 m, 3H; 8,17 m, 6H; 10,84 s, 1H.

Point de fusion : 100°C

**Exemple 19 - Chlorhydrate du N-{6-[3-(2-amino-éthyl)-1H-indol-5-yloxy]-hexyl}- méthane sulfonamide**

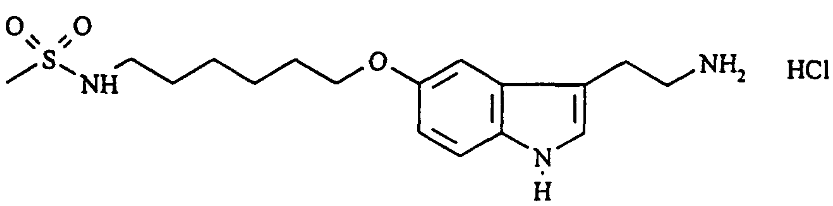

**19**

Le produit 18A (900 mg; 2,39 mmol) en solution dans le dichlorométhane (25 ml) en présence de triéthylamine (500 µl; 3,59 mmol) est traité, à température ambiante et sous azote, par la chlorure de mésyle (223 µl; 2,87 mmol). Après une heure d'agitation, le milieu est dilué au dichlorométhane et lavé à l'eau. La phase organique est séchée sur sulfate de sodium, filtrée et évaporée à sec.

Le produit brut obtenu est repris dans le dichlorométhane (15ml) et traité par une solution d'acide chlorhydrique dans l'éther en présence de quelques gouttes d'eau pendant 12 heures. Cette méthode permet d'obtenir directement le composé 19 sous la forme de cristaux verts pâles (743 mg; 80%).

Analyse élémentaire ($C_{17}H_{28}N_3O_3SCl$), % calculés : C 52,36; H 7,24; N 10,78; Cl 9,09; % trouvés : C 52,07; H 7,20; N 10,60; Cl 9,69.

RMN $^1H$, DMSO-d6 (ppm) : 1,41-1,73 m, 8H; 2,88-2,99 m, 9H; 3,97 t, 2H; 6,72 dd, 1H; 6,97-7,26 m, 4H; 8,07 s, 3H; 10,82 s, 1H.

Point de fusion : 160°C.

**Exemple 20 - Chlorhydrate du 6-[3-(2-amino-éthyl)-1H-indol-5-yloxy]-N-éthylhexanamide**

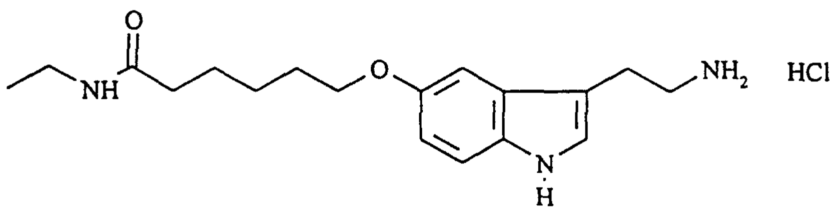

**20**

Le composé 20 est préparé à partir du 6-bromo-N-éthylhexanamide (362 mg; 1,63 mmol) et du composé 1A (300 mg; 1,09 mmol) selon la méthode décrite pour la préparation de l'exemple 4.

Le produit obtenu est déprotégé dans les conditions décrites pour la préparation de l'exemple 19. Le composé 20 est ainsi obtenu sous la forme d'une poudre blanche (217 mg; 56%).

Analyse élémentaire ($C_{18}H_{21}N_3O_2Cl$, 0,5 $H_2O$), % calculés : C 59,58; H 8,05; N 11,58; Cl 9,77; % trouvés : C 59,52; H 7,84; N 11,32; Cl 11,13

RMN $^1H$, DMSO-d6 (ppM) : 0,96 t, 3H; 1,37-1,72 m, 6H; 2,05 t, 2H; 2,98 m, 6H; 3,92 t, 2H; 6,67 dd, 1H, 7,03-7,23 m, 3H; 7,81 s, 1H; 8,05 s, 3H; 10,79 s, 1H.

Point de fusion : 195°C

**Exemple 21 - Chlorhydrate du 5-[3-(2-amino-éthyl)-1H-indol-5-yloxy]-pentanoate d'isopropyle**

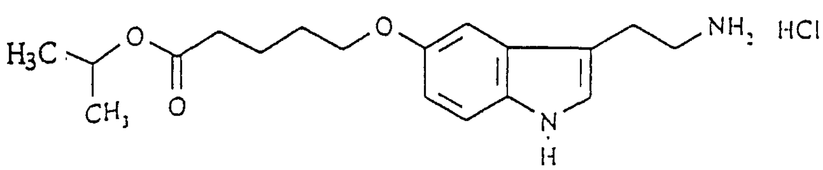

**21**

Le composé 21 est préparé à partir du 5-bromopentanoate d'isopropyle (727 mg; 3,26 mmol) et du composé 1A (500 mg; 1,81 mmol) selon la méthode décrite pour la préparation de l'exemple 1.

Le sirop obtenu est chromatographié sur colonne de gel de silice éluée par un mélange dichlorométhane/méthanol/ammoniaque (85:14:1; v/v). Le produit pur est isolé sous forme de sirop jaune qui conduit, après traitement à l'acide chlorhydrique dans l'éther, au composé 21 (159 mg; 25%).

Analyse élémentaire ($C_{18}H_{27}N_2O_3Cl$), % calculés : C 60,94; H 7,67; N 7,89; Cl 9,99; % trouvés : C 61,06; H 7,80; N 7,57; Cl 9,50.

RMN$^1H$, DMSO-d6 (ppm) : 1,16 d, 6H; 1,71 m, 4H; 2,33 t, 2H; 2,98 m, 4H; 3,96 t, 2H; 4,89 m, 1H; 6,71 dd, 1H; 7,05-7,26 m, 3H; 7,96 s, 3H; 10,80 s, 1H.

Point de fusion : 130°C.

**Exemple 22 - Chlorhydrate du 5-[3-(2-amino-éthyl)-1H-indol-5-yloxy]-pentanoate de benzyle**

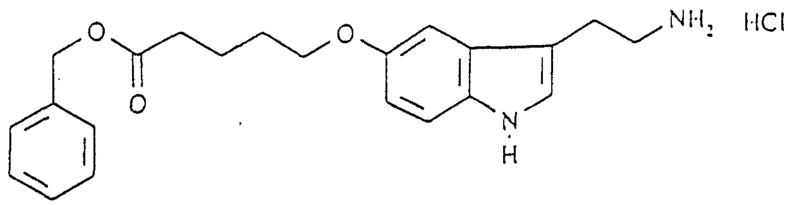

22

Le composé 22 est préparé à partir du 5-bromopentanoate de benzyle (883 mg. 3,25 mmol) et du composé 1A (500 mg; 1,81 mmol) selon la méthode décrite pour la préparation de l'exemple 1.

Le sirop obtenu est chromatographié sur colonne de gel de silice éluée par un mélange dichlorométhane/méthanol/ammoniaque (85:14:1; v/v). Le produit pur est isolé sous forme de sirop incolore qui conduit, après traitement à l'acide chlorhydrique dans l'éther, au composé 22 (449 mg; 62%).

Analyse élémentaire ($C_{22}H_{27}N_2O_3Cl$), % calculés : C 65,58; H 6,75; N 6,95; Cl 8,79; % trouvés : C 65,15; H 6,77; N 6,90; Cl 8,92

RMN $^1H$, DMSO-d6 (ppm) : 1,72 m, 4H; 2,44 m, 2H; 2,99 m, 4H; 3,96 m, 2H, 6,69 dd, 1H; 7,06-7,34 m, 8H; 8,08 s, 3H; 10,83 s, 1H.

Point de fusion : 162°C

**Exemple 23 - Chlorhydrate de l'acétate de 4-[3-(2-amino-éthyl)-1H-indol-5-yloxy]-butyle**

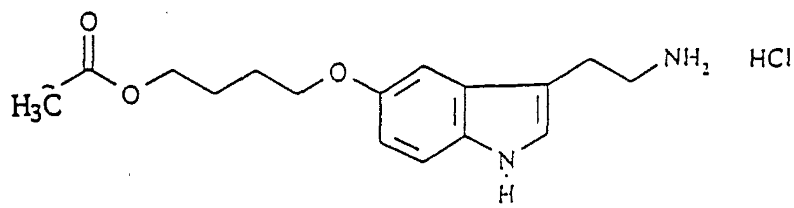

23

***23A*** *- Acétate de 4-[3-(2-N-(terbutoxycarbonyl)-amino-éthyl)-1H-indol-5-yloxy]-butyle*

Le composé 23A est obtenu à partir du 4-bromoacétate de butyle (0,94 ml; 6,5 mmol) et du composé 1A (1 g; 3,62 mmol) selon la méthode décrite pour la préparation du produit 1B.

Le sirop obtenu est purifié sur colonne de gel de silice éluée par un mélange dichlorométhane/acétone (20:1, v/v). Le produit pur est isolé sous forme de sirop jaune pâle (1,26 g; 89%).

***23*** *- Chlorhydrate de l'acétate de 4-[3-(2-amino-éthyl)-1H-indol-5-yloxy]-butyle*

Le composé 23A (508 mg; 1,30 mmol) est ensuite déprotégé selon la méthode décrite pour la préparation de l'exemple 1 à partir de 1B.

Le sirop obtenu est chromatographié sur colonne de gel de silice éluée par un mélange dichlorométhane/méthanol/ammoniaque (80:18,5:1,5; v/v). Le produit pur est isolé sous forme de sirop incolore qui conduit, après traitement à l'acide chlorhydrique dans l'éther, au composé 23 (309 mg; 73%).

Analyse élémentaire ($C_{16}H_{23}N_2O_3Cl$), % calculés : C 58,81; H 7,09; N 8,57; Cl 10,85; % trouvés : C 58,71; H 7,11; N 8,41; Cl 10,59

RMN $^1H$, DMSO-d6 (ppm) : 1,76 m, 4H; 2,01 s, 3H; 2,98 m, 4H; 3,99-4,08 m, 4H; 6,72 dd, 1H; 7,07-7,27 m, 3H; 7,99 s, 3H; 10,81 s, 1H

Point de fusion : 171°C

**Exemple 24 - Chlorhydrate du 4-[3-(2-amino-éthyl)-1H-indol-5-yloxy]-butan-1-ol**

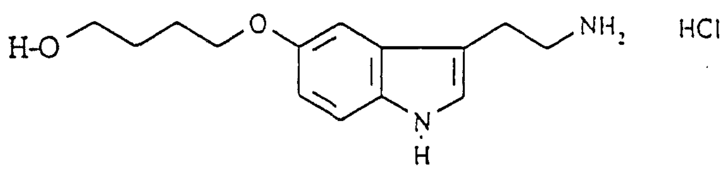

24

Le composé 23A (706 mg; 1,81 mmol) en solution dans l'éthanol (12,5 ml) est traité par la potasse (203 mg; 3,62 mmol), à température ambiante, pendant 4h30. Le milieu est alors dilué à l'eau et le pH est ramené vers 2-3 par addition d'une solution normale d'acide chlorhydrique. Ce mélange est ensuite extrait à l'acétate d'éthyle; la phase organique est séchée sur sulfate de sodium, filtrée et évaporée à sec. Le produit brut obtenu est ensuite déprotégé selon la méthode décrite pour la préparation de l'exemple 1 à partir de 1B.

Le sirop obtenu est chromatographié sur colonne de gel de silice éluée par un mélange dichlorométhane/méthanol/ammoniaque (85:14:1; v/v). Le produit pur est isolé sous forme de sirop jaune pâle qui conduit, après traitement à l'acide chlorhydrique dans l'éther, au composé 24 (252 mg; 50%).

Analyse élémentaire ($C_{14}H_{21}N_2O_2Cl$, 0,2 $H_2O$) % calculé : C 58,31; H 7,48; N 9,71 Cl 12,29; % trouvés : C 58,15; H 7,19; N 9,49; Cl 12,26.

RMN $^1H$, DMSO-d6 (ppm) : 1,59-1,77 m, 4H; 3,00 m, 4H; 3,49 m, 2H; 3,99 t, 2H; 4,49 t, 1H; 6,73 d, 1H; 7,06-7,28 m, 3H; 7,93 s, 3H; 10,81 s, 1H.

Point de fusion : 184°C

**Exemple 25 - Chlorhydrate du 5-[3-(2-amino-éthyl)-1H-indol-5-yloxy]-N-éthylpentanamide.**

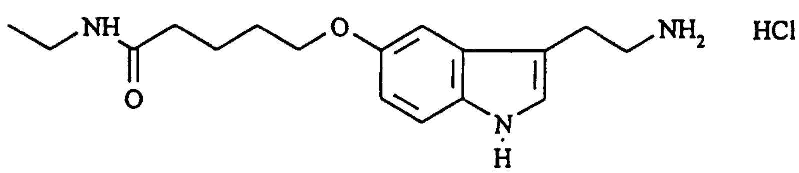

25

Le composé 25 est préparé à partir du 5-bromo-N-éthylpentanamide (339 mg; 1,62 mmol) et du composé 1A (300 mg; 1,09 mmol) selon la méthode décrite pour la préparation de l'exemple 4.

Le sirop obtenu est chromatographié sur colonne de gel de silice éluée par un mélange dichlorométhane/méthanol/ammoniaque (80:18:2; v/v). Le produit pur est isolé sous forme de sirop jaune pâle qui conduit, après traitement à l'acide chlorhydrique dans l'éther, au composé 25 (179 mg; 48%).

Analyse élémentaire ($C_{17}H_{26}N_3O_2Cl$, 0,4 $H_2O$), % calculés : C 58,83; H 7,78; N 12,11; Cl 10,21; % trouvés : C 58,84; H 7,56; N 11,81; Cl 11,07

RMN $^1H$, DMSO-d6 (ppm) : 0,97 t, 3H; 1,65 m, 4H; 2,09 t, 2H; 2,96 m, 6H; 3,93 m, 2H; 6,68 dd, 1H; 7,04-7,23 m, 3H; 7,84 s, 1H; 8,03 s, 3H; 10,78 s, 1H

Point de fusion : 184°C

**Exemple 26 - Chlorhydrate du 5-[3-(2-amino-éthyl)-1H-indol-5-yloxy]-pentanenitrile**

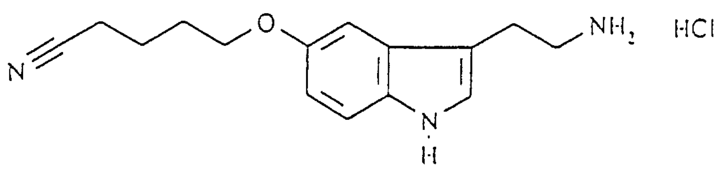

*26A - 5-{3-[2-N-(terbutoxycarbonyl)-amino-éthyl]-1H-indol-5-yloxy}-pentanenitrile.*

Le composé 26A est préparé à partir du 5-bromovaleronitrile (1,9 ml; 16,29 mmol) et du composé 1A selon la procédure décrite pour la préparation du produit 1B.

Le sirop obtenu est chromatographié sur colonne de gel de silice éluée par un mélange dichlorométhane/acétone (40:1; v/v). Le produit pur est isolé sous forme de sirop jaune pâle (2,94 g; 91%).
RMN $^1$H, DMSO-d6 (ppm) : 1,35 s, 9H; 1,74 m, 4H; 2,56 t, 2H; 2,72 t, 2H; 3,14 m, 2H; 3,97 t, 2H; 6,68 dd, 1H; 6,82-7,32 m, 4H; 10,60 s, 1H.

*26 - Chlorhydrate du 5-[3-(2-amino-éthyl)-1H-indol-5-yloxy]-pentanenitrile.*

Le composé 26A (600 mg; 1,67 mmol) est ensuite déprotégé selon la méthode décrite pour la préparation de l'exemple 1 à partir de 1B.

Le sirop obtenu est chromatographié sur colonne de gel de silice éluée par un mélange dichlorométhane/méthanol/ammoniaque (85:14:1; v/v). Le produit pur est; isolé sous forme de sirop incolore qui conduit, après traitement à l'acide chlorhydrique dans l'éther, au composé 26 (304 mg; 62%).
Analyse élémentaire ($C_{15}H_{20}N_3O_1Cl$), % calculés : C 61,32; H 6,86; N 14,30; Cl 12,07; % trouvés : C 60,87; H 6,77; N 13,96; Cl 11,37
RMN $^1$H, DMSO-d6 (ppm) : 1,78 m, 4H; 2,61 t, 2H; 3,01 m, 4H; 4,02 t, 2H; 6,74 dd, 1H; 7,10-7,28 m, 3H; 8,06 s, 3H; 10,83 s, 1H.
Point de fusion : 182°C

**Exemple 27 - Chlorhydrate du 5-[3-(2-amino-éthyl)-1H-indol-5-yloxy]-pentylamine**

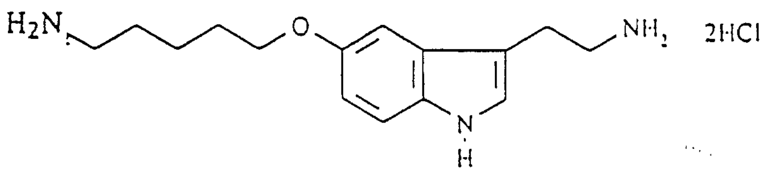

27

*27A - 5-{3-[2-N-(terbutoxycarbonyl)-amino-éthyl]-1H-indol-5-yloxy}-pentylamine*

Le composé 26A (2,34 g; 6,55 mmol) en solution dans le tétrahydrofurane (88 ml), en présence d'ammoniaque (5,9 ml) et de Nickel de Rancy (2 spatules), est soumis à une pression atmosphérique d'hydrogène pendant 26 heures.

Le mélange est alors filtré sur célite et le solvant évaporé à sec. Le sirop obtenu est purifié sur colonne de gel de silice éluée par un mélange dichlorométhane/méthanol/ ammoniaque (80:18,5:1,5; v/v).

Le produit pur est obtenu sous forme de sirop incolore (1,27 g; 54%).
RMN $^1$H, DMSO-d6 (ppm) : 1,40-1,70 m, 15H; 2,56-3,25 m, 6H; 3,97 t, 2H; 6,70 dd, 1H; 7,01-7,28 m, 4H; 10,58 s, 1H.

*27 - Chlorhydrate du 5-[3-(2-amino-éthyl)-1H-indol-5-yloxy]-pentylamine*

Le produit 27A (522 mg; 1,44 mmol) est déprotégé selon la méthode décrite pour la préparation de l'exemple 1 à partir de 1B.

Le sirop obtenu est chromatographié sur colonne de gel de silice éluée par un mélange dichlorométhane/méthanol/ammoniaque (75:20:5; v/v). Le produit pur est isolé sous forme de cristaux blancs qui conduisent, après traitement à l'acide chlorhydrique dans l'éther, au composé 27 (307 mg; 62%).
Analyse élémentaire ($C_{15}H_{25}N_3O_1Cl_2$), % calculés : C 52,21; H 7,65; N 12,18; Cl 20,55; % trouvés : C 52,07; H 7,30; N 11,83; Cl 19,27
RMN $^1H$, DMSO-d6 (ppm) : 1,48-1,73 m, 6H; 2,78 t, 2H: 2,98 m, 4H; 3,97 t, 2H; 6,71 dd, 1H; 7,09-7,26 m, 3H; 7,81 large s, 6H; 10,83 s, 1H.
Point de fusion : 123°C

**Exemple 28 - Chlorhydrate du N-{5-[3-(2-amino-éthyl)-1H-indol-5-yloxy]-pentyl}-méthanesulfonamide**

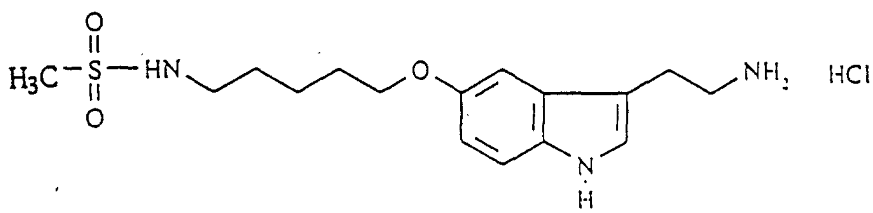

28

Le produit 28 est préparé à partir du composé 27A (559 mg; 1,54 mmol) selon la méthode décrite pour la préparation de l'exemple 19.

Le produit brut est ensuite déprotégé selon la méthode décrite pour la préparation de l'exemple 1 à partir de 1B.

Le sirop obtenu est chromatographié sur colonne de gel de silice éluée par un mélange dichlorométhane/méthanol/ammoniaque (80:18,5:1,5; v/v). Le produit pur est isolé sous forme de sirop jaune pâle qui conduit, après traitement à l'acide chlorhydrique dans l'éther, au composé 28 (396 mg; 68%).
Analyse élémentaire ($C_{16}H_{26}N_3O_3S_1Cl$), % calculés : C 51,12; H 6,97; N 11,18; Cl 9,43; % trotivés : C 51,23; H 6,97; N 10,78; Cl 9,69
RMN $^1H$, DMSO-d6 (ppm) : 1,52 m, 4H; 1,71 m, 2H; 2,88-2,99 m, 9H; 3,97 t, 2H; 6,72 dd, 1H; 6,98-7,27 m, 4H; 8,02 s, 3H; 10,81 s, 1H.
Point de fusion : 147°C

**Exemple 29 - Chlorhydrate du (±)-[3-(2-amino-éthyl)-1H-indol-5-yloxy]-phényl-acétate de méthyle**

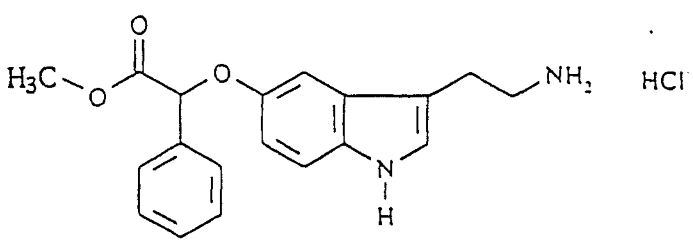

29

*29A - (±)-{3-[2-N-(terbutoxycarbonyl)-amino-éthyl]-1H-indol-5-yloxy}-phénylacétate de méthyle*

Le composé 29A est obtenu à partir du (±)-α-bromophénylacétate de méthyle (5,1 ml; 32,56 mmol) et du composé 1A (5 g; 18,09 mmol) suivant la méthode décrite pour la préparation du produit 1B.

Le sirop obtenu est chromatographié sur colonne de gel de silice éluée par un mélange hexane/acétate d'éthyle (2 :1 puis 1:1; v/v). Le produit pur est isolé sous forme de mousse rose (5,95 g; 77%).
RMN $^1H$, $CDCl_3$ (ppm) : 1,45 s, 9H; 2,87 t, 2H; 3,40 m, 2H; 3,75 s, 3H; 5,70 s, 1H; 6,85-7,66 m, 9H; 8,14 s, 1H.

*29 - Chlorhydrate du (±)-[3-(2-amino-éthyl)-1H-indol-5-yloxy]-phényl-acétate de méthyle*

Le composé 29A (500 mg; 1,18 mmol) est déprotégé selon la méthode décrite pour la préparation de l'exemple 1 à partir de 1B.

Le sirop obtenu est chromatographié sur colonne de gel de silice éluée par un mélange dichlorométhane/méthanol/ammoniaque (80:19.5:0,5; v/v). Le produit pur est isolé sous forme de sirop incolore qui conduit, après traitement à l'acide chlorhydrique dans l'éther, au composé 29 (237 mg; 60%).
Analyse élémentaire ($C_{19}H_{21}N_2O_3Cl$), % calculés : C 63,24; H 5,87; N 7,76; % trouvés : C 63,21; H 5,81; N 7,78
RMN $^1H$, DMSO-d6 (ppm) : 2,98 m, 4H; 3,65 s, 3H; 5,98 s, 1H; 6,82 dd, 1H; 7,11-7,62 m, 8H; 8,08 s, 3H; 10,89 s, 1H.
Point de fusion : 193°C (décomposition)

**Exemple 30 - Clilorhydrate du (±)-2-[3-(2-amino-éthyl)-1H-indol-5-yloxy]-2-phényl-éthanol.**

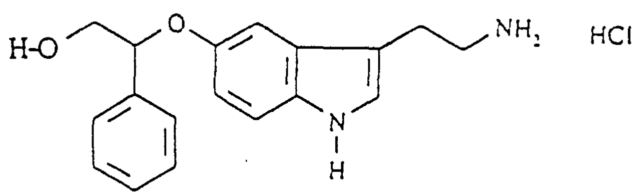

30

Le composé 29A (400 mg; 0,96 mmol) en solution dans le tétrahydrofurane anhydre (10 ml) est traité, sous azote et à 0°C, par l'hydrure d'aluminium lithium (72 mg; 1,92 mmol). Le milieu est agité à température ambiante pendant 3 heures. Le mélange est ensuite traité par un mélange sulfate de sodium / eau afin d'hydrolyser l'excès de réactif. La pâte formée est filtrée sur célite puis celle-ci est lavée au tétrahydrofurane et à l'acétate d'éthyle. Les solvants sont évaporés sous pression réduite pour conduire à un sirop brun. Ce sirop est purifié sur colonne de gel de silice éluée par un mélange dichlorométhane/acétone (15:1; v/v). Le produit pur est isolé sous forme de sirop brun (398 mg; 84%).

Ce sirop est ensuite repris dans le dichlorométhane (5 ml) et traité par une solution d'acide chlorhydrique dans l'éther en présence de quelques gouttes d'eau. Après 1 heure les cristaux blancs formés sont isolés pour conduire au composé 30 (150 mg; 46%).
Analyse élémentaire ($C_{18}H_{21}N_2O_2Cl$, 0,4 $H_2O$), % calculés : C 63,58; H 6,46; N 8,24; % trouvés : C 63,81; H 6,20; N 8,03
RMN $^1H$, DMSO-d6 (ppm) : 2,94 m, 4H; 3,57-3,83 m, 2H; 5,10 m, 1H; 6,74 dd, 1H, 7,04-7,46 m, 8H; 7,95 s, 3H; 10,76 s, 1H.
Point de fusion : 200°C

**Exemple 31 - Chlorhydrate de l'acide (±)-[3-(2-amino-éthyl)-1H-indol-5-yloxy]-phényl-acétique**

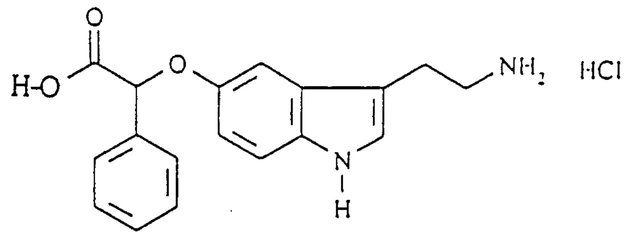

31

***31A*** *- Acide (±)-{3-[2-(terbutoxycarbonyl)-amino-éthyl]-1H-indol-5-yloxy}-phényl-acétique*

Le composé 29A (600 mg; 1,41 mmol) en solution dans l'éthanol (6 ml) est traité par la potasse (316 mg; 5,6 mmol) à température ambiante pendant 1h30.

Le milieu est ensuite dilué à l'acétate d'éthyle et lavé par une solution normale d'acide chlorhydrique jusqu'à pH 3. La phase organique est séchée sur sulfate de sodium, filtrée et évaporée à sec.

Le sirop obtenu est chromatographié sur colonne de gel de silice éluée par un mélange dichlorométhane/méthanol (5:1; v/v). Le produit pur est isolé sous forme de sirop donnant une mousse beige à l'évaporation (578 mg; 99%).
Analyse élémentaire ($C_{23}H_{26}N_2O_5$, 0,5 $H_2O$), % calculés : C 65,86; H 6,49; N 6,68; % trouvés : C 66,00; H 6,61; N 6,23
RMN $^1H$, $CDCl_3$ (ppm) : 1,42 s, 9H; 2,81 t, 2H; 3,35 m, 2H; 5,68 s, 1H; 6,90-7,67 m, 9H; 8,03 s, 1H

***31*** *- Chlorhydrate de l'acide (±)-[3-(2-amino-éthyl)-1H-indol-5-yloxy]-phényl-acétique*

Ce produit est repris dans le dichlorométhane (8 ml) et traité par une solution d'acide chlorhydrique dans l'éther en présence de quelques gouttes d'eau. Après 20mn les cristaux beiges formés sont isolés pour conduire au composé 31 (472 mg; 96%).
Analyse élémentaire ($C_{18}H_{19}N_2O_3Cl$, 0,5 $H_2O$), % calculés : C 60,76; H 5,67; N 5,87; % trouvés : C 60,63; H 5,65; N 7,48
RMN $^1H$, DMSO-d6 (ppm) : 2,97 m, 4H; 5,82 s, 1H; 6,80 dd, 1H; 7,15-7,62 m, 8H; 8,06 s, 3H; 10,88 s, 1H; 13,06 large s, 1H.
Point de fusion : 178°C

**Exemple 32 - Chlorhydrate du (±)-2-[3-(2-amino-éthyl)-1H-indol-5-yloxy]-2-phényl-acétamide**

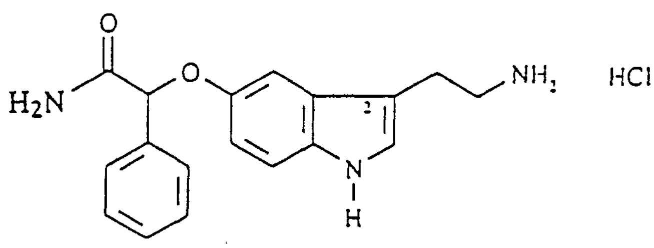

32

Le composé 29A (600 mg; 1,41 mmol) en solution dans l'éthanol (4 ml), en présence d'ammoniaque (6 ml) et de chlorure d'ammonium (1 g), est chauffé à 65°C pendant 17 heures. Après ce temps l'ammoniaque (2 ml) et le chlorure d'ammonium (1 g) sont de nouveau additionnés et le mélange est chauffé 20 heures de plus.

Le milieu est ensuite évaporé à sec; le sirop est repris dans l'acétate d'éthyle et lavé à l'eau puis par une

solution saturée de chlorure de sodium. La phase organique est séchée sur sulfate de sodium, filtrée et évaporée à sec. Le sirop obtenu est purifié sur colonne de gel de silice éluée par un mélange dichlorométhane/acétone (10:1; v/v). Le produit pur est isolé sous forme de mousse blanche (455 mg; 52%).

Ce produit est ensuite repris dans le dichlorométhane (6 ml) et traité par une solution d'acide chlorhydrique dans l'éther en présence de quelques gouttes d'eau. Après 20 mn les cristaux blancs formés sont isolés pour conduire au composé 32 (271 mg; 76%).
Analyse élémentaire ($C_{18}H_{20}N_3O_2Cl$, 0,8 $H_2O$), % calculés : C 60,02; H 6,04; N 11,66; % trouvés : C 60,10; H 5,83; N 11,42
RMN $^1H$, DMSO-d6 (ppm) : 3,01 m, 4H; 5,66 s, 1H; 6,84 dd, 1H; 7,18-7,87 m, 8H; 8,09 s, 3H; 10,86 d, 1H.
Point de fusion : 130°C

**Exemple 33 - Chlorhydrate du (±)-[3-(2-amino-éthyl)-1H-indol-5-yloxy]-phényl-acétate d'éthyle**

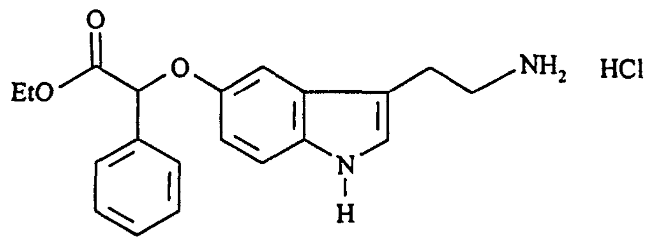

33

Le composé 31A (200 mg; 0,487 mmol) en solution dans l'éthanol (3 ml) est traité par une solution d'acide chlorhydrique dans l'éther (excès). Après 1 heure d'agitation à température ambiante le mélange est évaporé à sec et coévaporé deux fois avec du toluène (10 ml). Le solide obtenu est recristallisé dans l'éthanol pour conduire au composé 33 sous forme de poudre blanche (120 mg; 71%).
Analyse élémentaire ($C_{20}H_{23}N_2O_3Cl$), % calculés : C 64,08; H 6,18; N 7,47; % trouvés : C 63,68; H 6,14; N 7,42
RMN $^1H$, DMSO-d6 (ppm) : 1,12 t, 3H; 2,96 m, 4H; 4,12 q, 2H; 5,93 s, 1H; 6,82 dd, 1H; 7,14-7,61 m, 8H; 7,99 s, 3H; 10,89 s, 1H.
Point de fusion : 253°C

**Exemple 34 - Chlorhydrate du (+)-[3-(2-amino-éthyl)-1H-indol-5-yloxy]-phényl-acétate d'éthyle et chlorhydrate du (-)-[3-(2-amino-éthyl)-1H-indol-5-yloxy]-phényl-acétate d'éthyle**

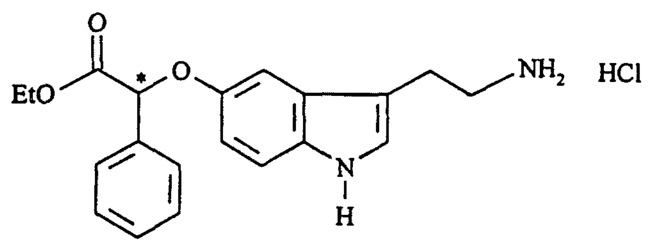

34

La séparation des deux énantiomères du composé 33 est réalisée par dérivatisation du composé 31A par le (R)-(-)-2-phénylglycinol, séparation des diastéréoisomères et formation des esters énantiomériquement purs.

Sur le composé 31A (1 g; 2,44 mmol) en solution dans le dichlorométhane (2,4 ml) sont additionnés successivement du PyBOP (1,40 g; 2;68 mmol), le (R)-(-)-2-phénylglycinol (367 mg; 2,68 mmol) et de la diisopropyléthylamine (0,64 ml; 3,66 mmol). Le mélange est agité à température ambiante pendant 2 heures puis évaporé à sec. Le sirop obtenu est repris dans le dichlorométhane, et lavé à l'eau; la phase organique est séchée sur sulfate de sodium, filtrée

et évaporée à sec.

Les deux diastéréoisomères obtenus sont séparés sur colonne de gel de silice éluée par un mélange dichlorométhane/acétate d'éthyle (4:1; v/v).

Chacun des deux produits obtenus est ensuite solubilisé dans un mélange éthanol/dioxanne (1:5; v/v) et traité par l'acide sulfurique concentré (excès) à 100°C pendant 1 heure.

Les milieux sont ensuite évaporés à sec, repris au dichlorométhane, lavés par une solution saturée de bicarbonate de soude puis à l'eau. Les phases organiques sont séchées sur sulfate de sodium, filtrées et évaporées à sec.

Les sirops obtenus sont purifiés sur colonnes de gel de silice éluées par un mélange chloroforme/méthanol/ammoniaque (80:18,5:1,5; v/v).

Le premier produit pur est isolé sous forme de sirop incolore qui conduit, après traitement à l'acide chlorhydrique dans l'éther, au composé 34A (105 mg; 24%).

Analyse élémentaire ($C_{20}H_{23}N_2O_3Cl$), % calculés : C 64,08; H 6,18; N 7,47; % trouvés : C 63,90; H 6,26; N 7,16

RMN $^1H$, DMSO-d6 (ppm) : 1,11 t, 3H; 2,96 m, 4H; 4,11 m, 2H; 5,92 s, 1H; 6,86 dd, 1H; 7,14-7,60 m, 8H; 7,93 large s, 3H; 10,89 s, 1H.

Point de fusion : 200°C

Pouvoir rotatoire : $[\alpha]_D$ + 62 (MeOH, c = 0,1)

Le second produit pur est isolé sous forme de sirop incolore qui conduit, après traitement à l'acide chlorhydrique dans l'éther, au composé 34B (51 mg; 12%).

Analyse élémentaire ($C_{20}H_{29}N_2O_3Cl$), % calculés : C 64,08; H 6,18; N 7,47; % trouvés : C 63,20; H 6,24; N 7,13

RMN $^1H$, DMSO-d6 (ppm) : 1,11 t, 3H; 2,97 m, 4H; 4,11 m, 2H; 5,92 s, 1H; 6,85 dd, 1H; 7,14-7,60 m, 8H; 8,06 s, 3H; 10,89 s, 1H.

Point de fusion : 225°C

Pouvoir rotatoire : $[\alpha]_D$ - 58° (MeOH; c = 0,12)

**Exemple 35 - Chlorhydrate du 5-[3-(1-méthyl-pipéridin-4-yl)-1H-indol-5-yloxy]-pentanoate d'éthyle**

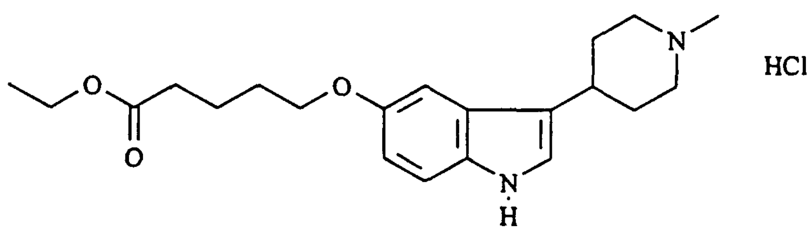

**35**

***35A -*** *3-(N-terbutoxycarbonyl-1,2,3,6-tétrahydro-pyridin-4-yl)-1H-indol-5-ol*

Sur une solution de sodium (4,8 g; 210 mmol) dans le méthanol sec (130 ml) sont ajoutés à température ambiante le 5-hydroxy-indole (4 g; 30,04 mmol) et la N-terbutoxycarbonyl-pipéridone (8,9 g; 45,1 mmol).

Le mélange est chauffé au reflux pendant 6 heures puis la solution est concentrée par évaporation sous pression réduite. Le sirop obtenu est repris dans le dichlorométhane, lavé par une solution molaire d'acide chlorhydrique puis à l'eau; la phase organique est séchée sur sulfate de magnésium, filtrée et évaporée à sec. Le sirop obtenu est chromatographié sur colonne de gel de silice éluée par un mélange dichlorométhane/acétone (10:1; v/v) puis dichlorométhane/méthanol (20;1; v/v). Le produit pur est isolé sou forme de poudre rose (6,35 g; 67%).

RMN $^1H$, DMSO-d6 (ppm) : 1,40 s, 9H; 2,46 m, 2H; 3,51 t, 2H; 3,99 m, 2H; 5,94 m, 1H; 6,58 dd, 1H; 7,10-7,29 m, 3H; 8,65 s, 1H; 10,84 s, 1H.

Point de fusion : 224-226°C

***35B -*** *3-(N-terbutoxycarbonyl-piperidin-4-yl)-1H-indol-5-ol*

Le composé 35A (5 g; 15,9 mmol) en solution dans le méthanol (100 ml) en présence d'une quantité catalytique d'oxyde de platine (4 spatules) est hydrogéné sous 40 psi dans un appareil de Parr pendant 1 nuit.

La solution est ensuite filtrée sur célite puis évaporée à sec. Le sirop obtenu est chromatographié sur colonne

de gel de silice éluée par un mélange dichlorométhane/acétone (10:1; v/v). Le produit pur est isolé sous forme de sirop incolore cristallisant dans l'éther pour donner une poudre blanche (4,17 g; 83%).
Analyse élémentaire ($C_{18}H_{24}N_2O_3$), % calculés : C 68,33; H 7,65; N 8,85; % trouvés : C 68,35; H 7,71; N 8,86
RMN $^1H$, DMSO-d6 (ppm) : 1,40-1,54 m, 11H; 1,84 m, 2H; 2,79 m, 3H; 4,00 m, 2H; 6,53 dd, 1H; 6,81 d, 1H; 6,96 d, 1H; 7,07 d, 1H; 8,53 s, 1H; 10,45 s, 1H.
Point de fusion : 170°C

***35C** - 3-(1-méthyl-pipéridin-4-yl)-1H-indol-5-ol*

Le composé 35B (1 g; 3,16 mmol) en solution dans le tétrahydrofurane anhydre (30 ml) est traité, sous azote et à température ambiante, par une solution molaire d' hydrure de lithium aluminium (6,3 ml; 6,32 mmol). Le mélange est chauffé à 65°C pendant 3 heures puis ramené à 0°C et traité par un mélange sulfate de sodium / eau. La pâte formée est filtrée sur célite et le filtrat est évaporé à sec pour donner une poudre blanche (625 mg; 86%). Ce produit est utilisé sans purification pour l'étape suivante.
RMN $^1H$, DMSO-d6 (ppm) : 1,58-2,07 m, 6H; 2,21 s, 3H; 2,58 m, 1H; 2,83 d, 2H; 6,59 dd, 1H; 6,84 d, 1H; 6,96 d, 1H; 7,09 d, 1H; 8,55 s, 1H; 10,44 s, 1H.

***35 -** Chlorhydrate du 5-[3-(1-méthyl-pipéndin-4-yl)-1H-indol-5-yloxy]-pentanoate d'éthyle*

Le composé 35C (120 mg; 0,52 mmol) en solution dans le diméthylformamide sec (3 ml) est traité par le carbonate de césium (255 mg; 0,78 mmol) à température ambiante pendant 15 minutes. Le 5-bromovalérate d'éthyle (100 µl; 0,62 mmol) est ensuite additionné et le mélange est agité à température ambiante pendant 4 heures. Le milieu est dilué à l'acétate d'éthyle et lavé à l'eau puis par une solution saturée de chlorure de sodium.
La phase organique est séchée sur sulfate de sodium, filtrée et évaporée à sec. Le sirop obtenu est chromatographié sur colonne de gel de silice éluée par un mélange dichlorométhane/méthanol/ammoniaque (85:14:1; v/v). Le produit pur est obtenu sous forme de solide jaune qui conduit, après traitement à l'acide chlorhydrique dans l'éther, au composé 35 (98 mg; 48%).
Analyse élémentaire ($C_{21}H_{31}N_2O_3Cl$, 1,5 $H_2O$), % calculés : C 59,78; H 8,12; N 6,64; Cl 8,40; % trouvés : C 59,98; H 7,88; N 6,43; Cl 9,00.
RMN $^1H$, DMSO-$d_6$ (ppm) : 1,18 t, 3H; 1,72 large s, 4H; 2,08 large s, 4H; 2,38 t, 2H; 2,78 s, 3H; 3,09 m, 3H; 3,45 m, 2H; 3,99-4,11 m, 4H; 6,71 dd, 1H; 7,09-7,26 m, 3H; 10,44 s, 1H; 10,74 s, 1H.

**Exemple 36 - Chlorhydrate du 5-(3-amino-1,2,3,4-tétrahydrocarbazole-6-yloxy)-pentanoate d'éthyle**

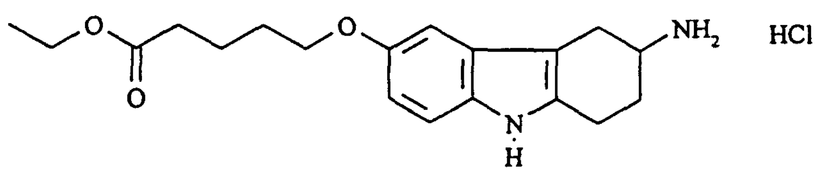

**36**

Le N-phtalimide du 3-amino-1,2,3,4-tétrahydro-6-hydroxycarbazole (Référence : G.E.A. COOMBES et al. J. Chem. Soc., n°2, 1970, 325-326) (1 g; 3,01 mmol) en solution dans le diméthylformamide (6 ml) en présence de carbonate de césium (1,47 g; 4,5 mmol) est traité par le 5-bromovalérate d'éthyle (0,86 ml; 5,4 mmol) à 70°C pendant une nuit.
Le milieu est dilué à l'acétate d'éthyle et lavé à l'eau puis par une solution saturée de chlorure de sodium. La phase organique est séchée sur sulfate de sodium, filtrée et évaporée à sec. Le sirop obtenu est chromatographié sur colonne de gel de silice éluée par un mélange dichlorométhane/acétone (100:1; v/v). Le produit pur est isolé sous forme de sirop vert (850 mg; 61%).
Ce produit (212 mg; 0,46 mmol) est déprotégé par chauffage à 90°C dans l'éthylène diamine (3,3 ml) pendant 2 heures. Le milieu est coévaporé 4 fois au toluène et les cristaux orangés obtenus sont ensuite purifiés sur colonne de gel de silice éluée par un mélange dichlorométhane/méthanol/ammoniaque (90:9:1; v/v). Le produit pur est isolé sous forme de cristaux jaunes qui conduisent, après traitement à l'acide chlorhydrique dans l'éther, au composé 36

(241 mg; 66%).
Analyse élémentaire : ($C_{19}H_{27}N_2O_3Cl$, 0,7 $H_2O$), % calculés : C 60,13; H 7,54; N 7,38; % trouvés : C 60,13; H 7,20; N 7,57.
RMN $^1H$, DMSO-d6 (ppm) : 1,16 t, 3H; 1,68 m, 4H; 1,91 m, 1H; 2,15 m, 1H; 2,35 t, 2H; 2,58-2,79 m, 3H; 3,03 dd, 1H; 3,44 m, 1H; 3,92 t, 2H; 4,02 q, 2H; 6,61 dd, 1H; 6,82 d, 1H; 7,10 d, 1H; 8,37 s, 3H; 10,66 s, 1H.
Point de fusion : 114°C

**Exemple 37 - Chlorhydrate du (R)-5-[3-(N-méthyl-pyrrolidin-2-ylméthyl)-1H-indol-5-yloxy]-pentanoate d'éthyle.**

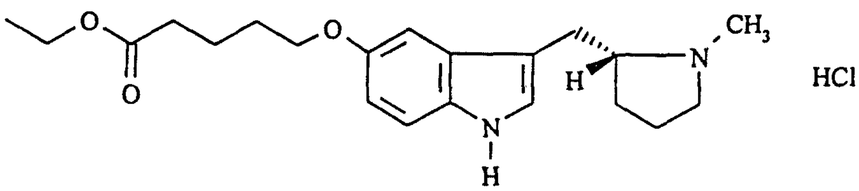

**37**

*37A - (R)-5-hydroxy-3-(N-méthyl-pyrrolidin-2-ylméthyl)-1H-indole*

Le (R)-5-méthoxy-3-(N-méthyl-pyrrolidin-2-ylméthyl)-1H-indole (Référence : J.E. Macor et al. : J. Med. Chem. 1992, 35, 4503-4505) (800 mg; 3,27 mmol) en solution dans le dichlorométhane anhydre (32 ml) est traité, sous azote et à -78°C, par une solution 1M de tribromure de bore (13 ml; 13,2 mmol). Le mélange est agité 2 heures à -78°C puis une heure à température ambiante puis ramenée à - 78°C pour être traitée par 4 ml d'éthanol. Le milieu est évaporé à sec et le sirop obtenu est purifié sur colonne de gel de silice éluée par un mélange dichlorométhane/ méthanol/ ammoniaque (85:14:1; v/v). Le produit pur est isolé sous forme de sirop incolore (429 mg; 57%).
RMN $^1H$, DMSO-d6 (ppm) : 1,45-1,62 m, 4H; 2,03 m, 1H; 2,34-2,43 m, 5H; 2,92 m, 2H; 6,55 dd, 1H; 6,79 d, 1H; 7,01 d, 1H; 7,08 d, 1H; 8,56 s, 1H; 10,44 s, 1H.

***37 -** Chlorhydrate du (R)-5-[3-(N-méthyl-pyrrolidin-2-ylméthyl)-1H-indol-5-yloxy]-pentanoate d'éthyle.*

Le composé 37A (250 mg; 1,08 mmol) en solution dans le diméthylformamide (2 ml) en présence de carbonate de césium (528 mg; 1,62 mmol) est traité par le 5-bromovalérate d'éthyle (0,31 ml; 1,9 mmol) à 60°C pendant une nuit.
Le mélange est filtrée sur célite et évaporé à sec. Le sirop obtenu est chromatographié sur colonne de gel de silice éluée par un mélange dichlorométhane/méthanol/ammoniaque (85:14:1; v/v). Le produit pur conduit, après traitement à l'acide chlorhydrique dans l'éther, au composé 37 (216 mg; 53%).

**Exemple 38 - Chlorhydrate du 5-[3-(2-{N-2-méthoxyéthyl}-éthyl)-1H-indol-5-yloxy]-N-éthylpentanamide.**

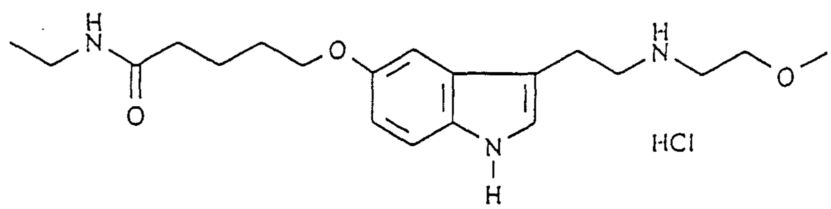

38

Le composé 25 (200 mg; 0,66 mmol), sous forme de base, en solution dans le dichlorométhane anhydre (4 ml) en présence de triéthylamine (0,138 ml ; 0,99; mmol) est traité à 0°C par le 2-bromométhoxyéthane ( 68 µl; 0,73 mmol). Le mélange est ensuite agité à température ambiante pendant 2 heures. Le milieu est dilué au dichlorométhane,

lavé à l'eau puis par une solution saturée de chlorure de sodium. La phase organique est séchée sur sulfate de sodium, filtrée et évaporée à sec. Le sirop obtenu est purifié sur colonne de gel de silice éluée par un mélange dichlorométhane/méthanol/ammoniaque (85:14:1;v/v). Le produit de monoalkylation est séparé du produit de dialkylation et conduit, après traitement à l'acide chlorhydrique dans l'éther, au composé 38 (60 mg; 25 %).

**Exemple 39 -Chlorhydrate du N-{6-[3-(2-N-méthyl-amino-éthyl)-1H-indol-5-yloxy]-hexyl}-méthane sulfonamide**

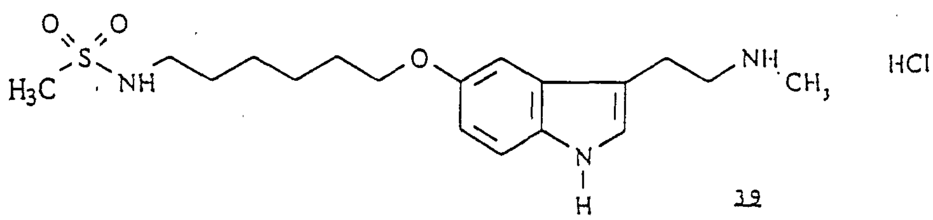

39

Le produit 19 sous sa forme protégé (-NHBOC) (300 mg ; 0.66 mmol) en solution dans le tétrahydrofurane anhydre (5 ml) est traité à température ambiante par une solution (1M) d'hydrure de lithium et d'aluminium dans le THF (2.64 ml ; 2.64 mmol). Le mélange est chauffé à 50°C pendant 4 heures puis ramené à température ambiante et neutralisé par addition d'un mélange sulfate de sodium/eau. Le milieu est filtré sur célite et le filtrat est évaporé à sec. Le sirop obtenu est chromatographié sur colonne de gel de silice éluée par un mélange dichlorométhane-méthanol-ammoniaque (80/18.5/1.5 ; v/v). Le produit pur est isolé sous forme d'un sirop qui conduit, après traitement à l'acide chlorhydrique dans l'éther, au composé 39 (93 mg ; 35 %).

**Exemple 40 - Chlorhydrate du N-{6-[3-(2-N-diméthyl-amino-éthyl)-1H-indol-5-yloxy]-hexyl-méthane sulfonamide**

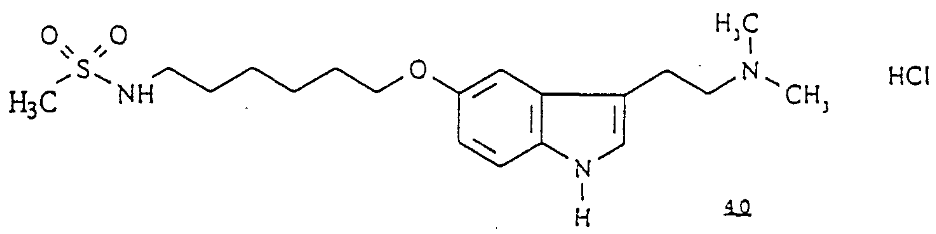

40

Sur le produit 19 (1.08 g ; 3.05 mmol) en solution dans le méthanol (43 ml) à - 4°C en présence de cyanoborohydrure de sodium (384 mg ; 6.11 mmol) et d'acide acétique glacial (0.87 ml ; 15.2 mmol) est additionnée goutte à goutte et en 10 minutes la formaldéhyde (38 % dans l'eau) (0.60 ml ; 7.64 mmol). Après 20 minutes d'agitation à 0°C et 1 h 20 à température ambiante une solution saturée de carbonate de potassium est additionnée puis le mélange est évaporé à sec. Le sirop est repris en dichlorométhane et lavé à l'eau. La phase organique est séchée sur sulfate de sodium, filtrée et évaporée à sec. Le sirop obtenu est chromatographié sur colonne de gel de silice éluée par un mélange dichlorométhane-méthanol-ammoniaque (90/9.5/0.5 ; v/v). Le produit pur est isolé sous forme de sirop incolore qui conduit, après traitement à l'acide chlorhydrique dans l'éther, au composé 40 sous forme de poudre blanche (880 mg ; 69 %).

| **Analyse élémentaire ($C_{19}H_{32}N_3O_35Cl$, 0.2EtOH) :** | | | | |
|---|---|---|---|---|
| **% calculés** | C = 54.54 ; | H = 7.83 ; | N = 9.84 ; | Cl = 8.30 |
| **% trouvés** | C = 54.73 ; | H = 7.84 ; | N = 9.67 ; | Cl = 8.40 |

RMN $^1H$, DMSO-$d_6$ (ppm) : 1.39-1.51 m, 6H ; 1.68-1.75 m, 2H ; 2.80 s, 6H ; 2.86 s, 3H ; 2.93 m, 2H ; 3.06-3.10 m, 2H ; 3.22-3.29 m, 2H ; 3.96 t. 2H ; 6.72 dd, 1H ; 6.97 t, 1H ; 7.14 dd, 2H ; 7,22 d, 1H ; 10.62 large s, 1H ; 10.81 s, 1H
Point de fusion : 160°C

## RESULTATS BIOLOGIQUES

Les récepteurs humains $5HT_{1D\alpha}$ et $5HT_{1D\beta}$ ont été clonés selon les séquences publiées par M. Hamblin et M. Metcalf, Mol. Pharmacol., **40,** 143 (1991) et Weinshenk et coll., Proc. Natl. Acad. Sci. **89,** 3630 (1992).

La transfection transitoire et la transfection permanente de gènes de ces récepteurs a été réalisée dans des lignées cellulaires Cos-7 et CHO-$K_1$ en utilisant un électroporateur.

La lignée cellulaire HeLa HA7 exprimant le récepteur $5HT_{1A}$ humain a été obtenue de Tulco (Duke Univ., Durham, N.C., USA) et cultivée selon la méthode de Fargin et coll., J. Biol. Chem. **264,** 14848 (1989).

L'étude de la liaison des dérivés de la présente invention avec les récepteurs $5HT_{1D\alpha}$, $5HT_{1D\beta}$ et $5HT_{1A}$ humains a été réalisée selon la méthode décrite par P. Pauwels et C. Palmier (Neuropharmacology, **33,** 67, 1994).

Les milieux d'incubation pour ces mesures de liaison comprennent 0,4 ml de préparation de membrane cellulaire, 0,05 ml d'un ligand tritié [[3H]-5CT (concentration finale : 2 nM) pour les récepteurs $5HT_{1D\alpha}$ et $5HT_{1D\beta}$ et [3H]-8OH-DPAT (concentration finale : 1 nM) pour le récepteur 5HT1A] et 0,05 ml de la molécule à tester (concentrations finales : 0,1 à 1000 nM) ou 10 µM (concentration finale) de sérotonine ($5HT_{1D\alpha}$ et $5HT_{1D\beta}$) ou 1 µM (concentration finale) de spiroxatrine (5HT1A).

**Résultats obtenus:** les quelques exemples qui suivent, choisis parmi les composés de la présente invention illustrent le profil des composés de la présente invention quant à leur liaison aux récepteurs $5HT_{1D}$ et $5HT_{1A}$ humains:

| | Ki (nM) | | |
|---|---|---|---|
| **exemples** | **$5HT_{1D\alpha}$** | **$5HT_{1D\beta}$** | **$5HT_{1A}$** |
| 16 | 1.5 | 0.7 | 9 |
| 17 | 2.2 | 2.6 | 24.3 |
| 18 | 2.4 | 2 | 78.6 |
| 19 | 1.1 | 0.7 | 11.5 |
| 20 | 2 | 1.1 | 46.5 |
| 23 | 0.9 | 0.4 | 40 |
| Sumatriptan | 9 | 26 | 440 |
| Naratriptan | 1.6 | 2 | 46 |

Les dérivés de la présente invention sont en outre capables, comme la sérotonine, d'induire la contraction des anneaux de veine saphène de lapin médiée par les récepteurs "$5HT_{1\text{-like}}$".

La technique mise en oeuvre a été adaptée de Van Heuven-Nolsen D. et al., Eur. J. Pharmacol. **191**, 375-382 (1990) et de Martin G.R. et Mc Lennan S.J., Naunyn-Schmiedeberg's Arch. Pharmacol. **342**, 111-119 (1990) et permet donc de déterminer une valeur de $pD_2$ et un $E_{max}$ relatif à la sérotonine pour chaque produit testé.

Les quelques exemples qui suivent, choisis parmi les composés de la présente invention, mettent en évidence leur profil agoniste dans ce modèle en comparaison avec le sumatriptan et le naratriptan.

| **Contraction de la veine saphène de lapin** | | |
|---|---|---|
| **exemples** | **pD2** | **$E_{max}$ relatif*** |
| 12 | 6.16 | 0.8 |
| 13 | 6.51 | 1.2 |
| 16 | 6.62 | 1.36 |
| 19 | 7.8 | 1 |
| Sumatriptan | 5.75 | 1.26 |
| Naratriptan | 5.54 | 1 |

* rapport entre le $E_{max}$ des composés cités et le $E_{max}$ de la sérotonine.

Ces résultats biologiques illustrent l'intérêt des composés de la présente invention, puisque, comme le montre les exemples ci-dessus, ils se comparent favorablement au sumatriptan et au naratriptan quant à leur liaison aux récepteurs $5HT_{1D}$ humains et à leur efficacité comme agonistes dans le modèle de la contraction de la veine saphène de lapin.

Les éthers aromatiques dérivés d'indoles faisant partie de cette invention sont des composés nouveaux ayant une très haute affinité et une très bonne sélectivité pour les récepteurs communément appelés $5HT_{1\text{-like}}$ et plus particulièrement pour les récepteurs appelés $5HT_{1B}$ et $5HT_{1D}$, selon la nouvelle nomenclature récemment proposée par P. Humphrey, P. Hartig et D. Hoyer (TIPS, 14, 233-236, 1993).

En thérapeutique humaine, les composés de formule générale (I) selon l'invention sont particulièrement utiles pour le traitement et la prévention des désordres liés à la sérotonine au niveau du système nerveux central et du système vasculaire. Ces composés peuvent donc être utilisés dans le traitement et la prévention de la dépression, des désordres compulsifs obsessionnels, des attaques de panique, de la boulimie et de l'anorexie, de l'agressivité, de l'alcoolisme, du tabagisme, de l'hypertension, de la nausée, du dysfonctionnement sexuel, du comportement asocial, de l'anxiété, de la migraine, de l'algie vasculaire de la face et des céphalées chroniques vasculaires, de la spasticité, de la maladie de Parkinson ou d'Alzheimer et des troubles de la mémoire.

La présente invention concerne également les médicaments constitués par au moins un composé de formule (I) à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale,nasale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine.

Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylène glycol, un polyéthylène glycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylène glycols.

Les compositions pour administration topique peuvent être par exemple des crèmes, lotions, collyres, collutoires, gouttes nasales ou aérosols.

Les doses dépendent de l'effet recherché, de la durée du traitement et de la voie d'administration utilisée ; elles sont généralement comprises entre 0,001 g et 1 g (de préférence comprises entre 0,005 g et 0,25 g) par jour de préférence par voie orale pour un adulte avec des doses unitaires allant de 0,1 mg à 500 mg de substance active, de préférence de 1 mg à 50 mg.

D'une façon générale, le médecin déterminera la posologie appropriée en fonction de l'âge, du poids et de tous les autres facteurs propres au sujet à traiter. Les exemples suivants illustrent des compositions selon l'invention [dans ces exemples, le terme "composant actif" désigne un ou plusieurs (généralement un) des composés de formule (I) selon la présente invention] :

Comprimés

On peut les préparer par compression directe ou en passant par une granulation au mouillé. Le mode opératoire par compression directe est préféré mais il peut ne pas convenir dans tous les cas selon les doses et les propriétés physiques du composant actif.

| *A - Par compression directe* | |
|---|---|
| | mg pour 1 comprimé |
| composant actif | 10,0 |
| cellulose microcristalline B.P.C. | 89,5 |
| stéarate de magnésium | 0,5 |
| | 100,0 |

On passe le composant actif au travers d'un tamis à ouverture de maille de 250 µm de côté, on mélange avec les excipients et on comprime à l'aide de poinçons de 6,0 mm. On peut préparer des comprimés présentant d'autres résistances mécaniques en modifiant le poids de compression avec utilisation de poinçons appropriés.

| *B - Granulation au mouillé* | |
|---|---|
| | mg pour un comprimé |
| composant actif | 10,0 |
| lactose Codex | 74,5 |
| amidon Codex | 10,0 |
| amidon de maïs prégélatinisé Codex | 5,0 |
| stéarate de magnésium | 0,5 |
| Poids à la compression | 100,0 |

On fait passer le composant actif au travers d'un tamis à ouverture de maille de 250 µm et on mélange avec le lactose, l'amidon et l'amidon prégélatinisé. On humidifie les poudres mélangées par de l'eau purifiée, on met à l'état de granulés, on sèche, on tamise et on mélange avec le stéarate de magnésium. Les granulés lubrifiés sont mis en comprimés comme pour les formules par compression directe. On peut appliquer sur les comprimés une pellicule de revêtement au moyen de matières filmogènes appropriées, par exemple la méthylcellulose ou l'hydroxy-propyl-méthylcellulose, selon des techniques classiques. On peut également revêtir les comprimés de sucre.

| Capsules | |
|---|---|
| | mg pour une capsule |
| composant actif | 10,0 |
| * amidon 1500 | 89,5 |
| stéarate de magnésium Codex | 0,5 |
| Poids de remplissage | 100,0 |

*une forme d'amidon directement compressible provenant de la firme Colorcon Ltd, Orpington, Kent, Royaume Uni.

On fait passer le composant actif au travers d'un tamis à ouverture de maille de 250 µm et on mélange avec les autres substances. On introduit le mélange dans des capsules de gélatine dure n°2 sur une machine à remplir appropriée. On peut préparer d'autres unités de dosage en modifiant le poids de remplissage et, lorsque c'est nécessaire, en changeant la dimension de la capsule.

Sirop

| | mg par dose de 5 ml |
|---|---|
| composant actif | 10,0 |
| saccharose Codex | 2750,0 |
| glycérine Codex | 500,0 |
| tampon ) | |
| arôme ) | |
| colorant ) | q.s. |
| préservateur ) | |
| eau distillée | 5,0 |

On dissout le composant actif, le tampon, l'arôme, le colorant et le préservateur dans une partie de l'eau et on ajoute la glycérine. On chauffe le restant de l'eau à 80°C et on y dissout le saccharose puis on refroidit. On combine les deux solutions, on règle le volume et on mélange. Le sirop obtenu est clarifié par filtration.

| Suppositoires | | |
|---|---|---|
| Composant actif | | 10,0 mg |
| * Witepsol H15 | complément à | 1,0 g |

* Marque commercialisée pour Adeps Solidus de la Pharmacopée Européenne.

On prépare une suspension du composant actif dans le Witepsol H15 et on l'introduit dans une machine appropriée avec moules à suppositoires de 1 g.

| Liquide pour administration par injection intraveineuse | | |
|---|---|---|
| | | g/l |
| composant actif | | 2,0 |
| eau pour injection Codex | complément à | 1000,0 |

On peut ajouter du chlorure de sodium pour régler la tonicité de la solution et régler le pH à la stabilité maximale et/ou pour faciliter la dissolution du composant actif au moyen d'un acide ou d'un alcali dilué ou en ajoutant des sels tampons appropriés. On prépare la solution, on la clarifie et on l'introduit dans des ampoules de dimension appropriée qu'on scelle par fusion du verre. On peut également stériliser le liquide pour injection par chauffage à l'autoclave selon l'un des cycles acceptables. On peut également stériliser la solution par filtration et introduire en ampoule stérile dans des conditions aseptiques. La solution peut être introduite dans les ampoules en atmosphère gazeuse.

| Cartouches pour inhalation | |
|---|---|
| | g/cartouche |
| composant actif micronisé | 1,0 |
| lactose Codex | 39,0 |

Le composant actif est micronisé dans un broyeur à énergie de fluide et mis à l'état de fines particules avant mélange avec du lactose pour comprimés dans un mélangeur à haute énergie. Le mélange pulvérulent est introduit en capsules de gélatine dure n°3 sur une machine à encapsuler appropriée. Le contenu des cartouches est administré à l'aide d'un inhalateur à poudre.

| Aérosol sous pression à valve doseuse | | |
|---|---|---|
| | mg/dose | pour 1 boîte |
| composant actif micronisé | 0,500 | 120 mg |
| acide oléique Codex | 0,050 | 12 mg |
| trichlorofluorométhane pour usage pharmaceutique | 22,25 | 5,34 g |
| dichlorodifluorométhane pour usage pharmaceutique | 60,90 | 14,62 g |

Le composant actif est micronisé dans un broyeur à énergie de fluide et mis à l'état de fines particules. On mélange l'acide oléique avec le trichlorofluorométhane à une température de 10-15°C et on introduit dans la solution à l'aide d'un mélangeur à haut effet de cisaillement le médicament micronisé. La suspension est introduite en quantité mesurée dans des boîtes aérosol en aluminium sur lesquelles on fixe des valves doseuses appropriées délivrant une dose de 85 mg de la suspension ; le dichlorodifluorométhane est introduit dans les boîtes par injection au travers des valves.

## Revendications

**1.** Composés répondant à la formule générale (I)

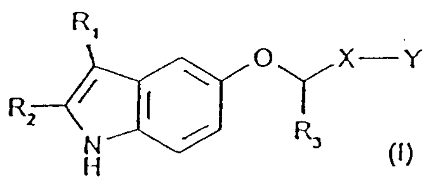

(I)

dans laquelle
R1 représente un reste aminé correspondant à l'une des formules (i) à (v) :

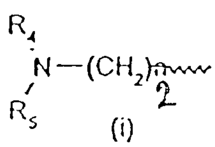

(i)

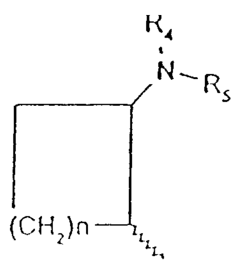

(ii)

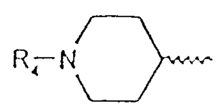

(iii)

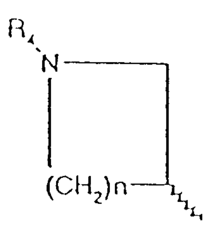

(iv)

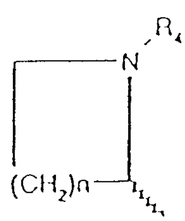

(v)

dans lesquelles n représente un nombre entier compris entre 1 et 5,
R4 représente un hydrogène, un groupement alkyle linéaire ou ramifié comprenant de 1 à 6 atomes de carbone ou un résidu du type $(CH_2)_mOR'$ dans lequel m représente un nombre entier compris entre 1 et 5, et R' un groupement alkyle linéaire ou ramifié comprenant de 1 à 6 atomes de carbone,
R5 représente un hydrogène, ou un groupement alkyle linéaire ou ramifié comprenant de 1 à 6 atomes de carbone,
$R_2$ représente un hydrogène, ou, $R_1$ et $R_2$, pris ensemble, forment un cycle à 6 atomes de carbone substitué par une fonction amine ($NR_4R_5$), à la condition que lorsque R1 représente un reste aminé de formule (i), $R_2$ représente un atome d'hydrogène,
$R_3$ représente un hydrogène, un résidu alkyle comprenant de 1 à 5 atomes de carbone ou un phényle,
X peut être omis ou représenter soit une chaîne alkyle linéaire ou ramifiée comprenant de 1 à 8 atomes de carbone, soit un phényle pouvant être diversement substitué en diverses positions par un groupe alkyle linéaire ou ramifié comprenant de 1 à 6 atomes de carbone, un phényle un halogène, un alcool (OH), un nitrile (CN), un nitro ($NO_2$), un thiol (SH),
Y représente un résidu carbonylé ($COR_6$), sulfonylé ($SO_2R_6$), oxygéné ($OR_7$), aminé ($NHR_8$), nitrile (CN), nitro ($NO_2$), oxime (C=NOH) ou hydroxylamine (NHOH) dans lesquels $R_6$ représente $R'_6$, $OR'_6$ ou $NHR''_6$ où $R'_6$ et $R''_6$ représentent une chaîne alkyle linéaire ou ramifiée de 1 à 8 atomes de carbone, un cycloalkyle de 4 à 10 atomes de carbone, un résidu aromatique tel qu'un phényle, un benzyle, ou un phénétyle, $R_7$ représente $R'_6$, $COR'_6$, $COOR'_6$ ou $CONHR'_6$ et $R_8$ représente un hydrogène ou un résidu tel que $R''_6$, $COR'_6$, $CO_2R'_6$, $CONHR'_6$, $SO_2R'_6$ ou $SO_2NR'_6R''_6$ avec les restrictions suivantes :

- lorsque Y représente $COR_6$, lorsque X est omis et lorsque $R_3$ représente un hydrogène, alors $R_1$ doit être différent de $CH_2CH_2N(R_4R_5)$.
- lorsque $R''_6$ représente un reste aromatique, alors $R_1$ doit être différent de $CH_2CH_2N(R_4R_5)$ et
- Y est différent d'un groupe alkoxy,

leurs sels, hydrates, solvats acceptables pour l'usage thérapeutique,
les composés de formule (I) contenant 1 ou plusieurs centres asymétriques présentent des formes isomères, les racémiques et les énantiomères purs de ces composés font également partie de cette invention.

**2.** Un composé de formule (I) selon la revendication 1, sélectionné parmi :

4-[3-(2-amino-éthyl)-1H-indol-5-yloxyméthyl]-benzoate d'éthyle.
4-[3-(2-amino-éthyl)-1H-indol-5-yloxyméthyl]-benzoate de méthyle.
{4-[3-(2-amino-éthyl)-1H-indol-5-yloxyméthyl]-phényl}-méthanol.
4-[3-(2-amino-éthyl)-1H-indol-5-yloxyméthyl]-benzoate de propyle.
2-[5-(4-nitro-benzyloxy)-1H-indol-3-yl]-éthylamine.
2-[5-(4-méthyl-3-nitro-benzyloxy)-1H-indol-3-yl]-éthylamine.
3-[3-(2-amino-éthyl)-1H-indol-5-yloxyméthyl]-benzoate de méthyle.
2-[5-(2-nitro-benzyloxy)-1H-indol-3-yl]-éthylamine.
2-[3-(2-amino-éthyl)-1H-indol-5-yloxyméthyl]-benzoate d'éthyle.
2-[3-(2-amino-éthyl)-1H-indol-5-yloxyméthyl] -1-phényl-éthanone.
2-[3-(2-amino-éthyl)-1H-indol-5-yloxy]-1-(4-méthoxy-phényl)-éthanone.
4-[3-(2-amino-éthyl)-1H-indol-5-yloxy)-butyrate d'éthyle.
5-[3-(2-amino-éthyl)-1H-indol-5-yloxy]-pentanoate d'éthyle.
6-[3-(2-amino-éthyl)-1H-indol-5-yloxy]-hexanoate d'éthyle.
6-[3-(2-amino-éthyl)-1H-indol-5-yloxy]-hexanoate de benzyle.
6-[3-(2-amino-éthyl)-1H-indol-5-yloxy]-hexanoate d'isopropyle.
6-[3-(2-amino-éthyl)-1H-indol-5-yloxy]-hexanitrile.
6-[3-(2-amino-éthyl)-1H-indol-5-yloxy]-hexylamine.
N-{6-[3-(2-amino-éthyl)-1H-indol-5-yloxy]-hexyl}-méthane sulfonamide.
6-[3-(2-amino-éthyl)-1H-indol-5-yloxy]-N-éthylhexanamide.
5-[3-(2-amino-éthyl)-1H-indol-5-yloxy]-pentanoate d'isopropyle.
5-[3-(2-amino-éthyl)-1H-indol-5-yloxy]-pentanoate de benzyle.
l'acétate de 4-[3-(2-amino-éthyl)-1H-indol-5-yloxy]-butyle.
4-[3-(2-amino-éthyl)-1H-indol-5-yloxy]-butan-1-ol.
5-[3-(2-amino-éthyl)-1H-indol-5-yloxy]-N-éthylpentanamide.
5-[3-(2-amino-éthyl)-1H-indol-5-yloxy]-pentanenitrile.
5-[3-(2-amino-éthyl)-1H-indol-5-yloxy]-pentylamine.
N-{5-[3-(2-amino-éthyl)-1H-indol-5-yloxy]-pentyl}-méthanesulfonamide.
(±)-[3-(2-amino-éthyl)-1H-indol-5-yloxy]-phényl-acétate de méthyle.
(±)-2-[3-(2-amino-éthyl)-1H-indol-5-yloxy]-2-phényl-éthanol.

(±)-[3-(2-amino-éthyl)-1H-indol-5-yloxy]-phényl-acétique.
(±)-2-[3-(2-amino-éthyl)-1H-indol-5-yloxy]-2-phényl-acétamide.
(±)-[3-(2-amino-éthyl)-1H-indol-5-yloxy]-phényl-acétate d'éthyle.
(+)-[3-(2-amino-éthyl)-1H-indol-5-yloxy]-phenyl-acétate d'éthyle.
(-)-[3-(2-amino-éthyl)-1H-indol-5-yloxy]-phényl-acétate d'éthyle.
5-[3-(1-méthyl-pipéridin-4-yl)-1H-indol-5-yloxy]-pentanoate d'éthyle.
5-(3-amino-1,2,3,4-tétrahydrocarbazole-6-yloxy)-pentanoate d'éthyle.
(R)-5-[3-(N-méthyl-pyrrolidin-2-ylméthyl)-1H-indol-5-yloxy]-pentanoate d'éthyle,

leurs sels, hydrates, solvats acceptables pour l'usage thérapeutique.

3. Composés selon la revendication 1 **caractérisés en ce que** $R_1$ représente

$$R_4R_5N\text{-}CH_2\text{-}CH_2\text{-}$$

4. Composés selon la revendication 1 **caractérisés en ce que** $R_4$ et $R_5$ représentent un hydrogène ou un méthyle.

5. Composés selon la revendication 1 **caractérisés en ce que** $R_2$ représente un hydrogène.

6. Composés selon la revendication 1 **caractérisés en ce que** X représente une chaîne alkyle linéaire ou ramifiée comprenant de 1 à 8 atomes de carbone.

7. Composés selon la revendication 1 **caractérisés en ce que** X représente un phényle sur lequel le substituant Y est attaché en position para.

8. Composés selon la revendication 1 **caractérisés en ce que** Y représente $COR_6$ ou $OCOR'_6$.

9. Composés selon la revendication 1 **caractérisés en ce que** Y représente $NHSO_2R'_6$.

10. Composés selon l'une des revendications 1 à 9 à l'état de sel acceptable pour l'usage thérapeutique **caractérisés en ce que** ces sels sont des chlorhydrates, bromhydrates, sulfates, méthanesulfonates, fumarates, maléates ou succinates.

11. Procédé de préparation des composés de formule (I) selon la revendication 1, **caractérisé en ce que** l'on fait réagir un intermédiaire de formule générale (II)

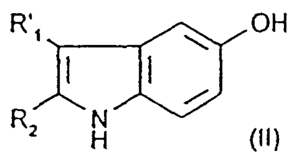

(II)

dans laquelle $R_2$ est défini comme précédemment et $R'_1$ peut être équivalent à $R_1$ ou à un précurseur de $R_1$ ( qui sera restauré, en fin de synthèse, par une réaction appropriée telle que par exemple la coupure d'un groupe protecteur) avec un dérivé de formule générale (III).

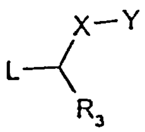

**(III)**

dans laquelle X, Y et $R_3$ sont définis comme précédemment et L représentent un groupe partant tel qu'un halogène

(iode, brome ou chlore), un mésylate, un tosylate ou un sulfate.

**12.** Procédé de préparation des composés de formule (I) selon la revendication 1, **caractérisé en ce que** l'on convertit un composé de formule générale (I) ou un sel ou un dérivé comportant un groupe protecteur d'un tel composé, en un autre composé de formule générale (I) selon le revendication 1.

**13.** Compositions pharmaceutiques contenant, à titre d'ingrédients actifs, un composé selon l'une des revendications 1 à 10, en combinaison avec un véhicule pharmaceutique acceptable comme médicaments.

**14.** Compositions pharmaceutiques contenant, à titre d'ingrédients actifs, un composé selon l'une des revendications 1 à 10, en combinaison avec un véhicule pharmaceutique acceptable, pour le traitement tant curatif que préventif des désordres liés à la sérotonine.

**15.** Compositions pharmaceutiques contenant, à titre d'ingrédients actifs, un composé selon l'une des revendications 1 à 10, en combinaison avec un véhicule pharmaceutique acceptable, pour le traitement tant curatif que préventif de la migraine, de l'algie vasculaire de la face et des céphalées chroniques vasculaires.

**16.** Compositions pharmaceutiques contenant, à titre d'ingrédients actifs, un composé selon l'une des revendications 1 à 10, en combinaison avec un véhicule pharmaceutique acceptable, pour le traitement tant curatif que préventif de la dépression, des désordres compulsifs obsessionnels, de l'anxiété et des attaques de panique.

**17.** Compositions pharmaceutiques contenant, à titre d'ingrédients actifs, un composé selon l'une des revendications 1 à 10, en combinaison avec un véhicule pharmaceutique acceptable, pour le traitement tant curatif que préventif de la schizophrénie, de la spasticité, de l'agressivité et/ou de l'alcoolisme et/ou du comportement asocial, des désordres alimentaires tels que la boulimie et l'anorexie, des dysfonctionnements sexuels.

**18.** Compositions pharmaceutiques contenant, à titre d'ingrédients actifs, un composé selon l'une des revendications 1 à 10, en combinaison avec un véhicule pharmaceutique acceptable, pour le traitement tant curatif que préventif et des maladies neurodégénératives telles que par exemple la maladie de Parkinson ou la maladie d'Alzheimer.

**Patentansprüche**

**1.** Verbindungen der allgemeinen Formel (I)

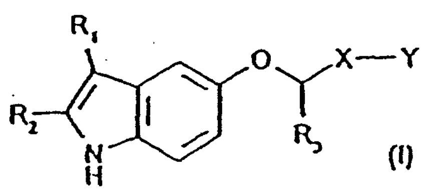

(I)

in der

$R_1$ einen aminierten Rest darstellt, der einer der Formeln (i) bis (v) entspricht:

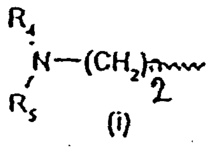

(i)

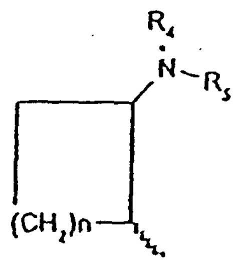

(ii)

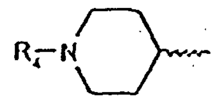

(iii)

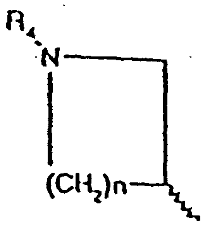

(iv)

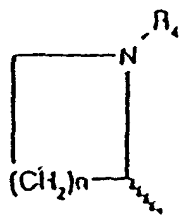

(v)

in denen n eine ganze Zahl von 1 bis 5 darstellt,

$R_4$ Wasserstoff, eine lineare oder verzweigte Alkylgruppe, die 1 bis 6 Kohlenstoff-Atome enthält, oder einen Rest der Art $(CH_2)_mOR'$ darstellt, in welchem m eine ganze Zahl von 1 bis 5 darstellt und R' eine lineare oder verzweigte Alkylgruppe ist, die 1 bis 6 Kohlenstoffatome enthält,

$R_5$ Wasserstoff oder eine lineare oder verzweigte Alkylgruppe, die 1 bis 6 Kohlenstoffatome enthält, darstellt,

R2 Wasserstoff darstellt, oder $R_1$ und $R_2$ zusammen genommen einen Cyclus mit 6 Kohlenstoff-Atomen bilden, der durch eine Aminfunktion ($NR_4R_5$) substituiert ist,

mit der Bedingung, dass, wenn $R_1$ einen aminierten Rest der Formel (i) darstellt, $R_2$ ein Wasserstoff-Atom darstellt,

R3 Wasserstoff, einen Alkylrest, der 1 bis 5 Kohlenstoff-Atome enthält, oder Phenyl darstellt,

X fehlen oder entweder eine lineare oder verzweigte Alkylkette, die 1 bis 8 Kohlenstoff-Atome enthält, oder Phenyl darstellen kann, das verschieden in verschiedenen Positionen durch eine lineare oder verzweigte Alkylgruppe, die 1 bis 6 Kohlenstoff-Atome enthält, ein Phenyl, ein Halogen, einen Alkohol (OH), ein Nitril (CN), ein Nitro ($NO_2$), ein Thiol (SH) substituiert sein kann,

Y einen carbonylierten ($COR_6$), sulfonylierten ($SO_2R_6$), oxygenierten ($OR_7$), aminierten ($NHR_8$) Rest, Nitril (CN), Nitro ($NO_2$), Oxim (C=NOH) oder Hydroxylamin (NHOH) darstellt, worin $R_6$ für $R'_6$, $OR'_6$ oder $NHR''_6$ steht, worin $R'_6$ und $R''_6$ eine lineare oder verzweigte Alkylkette mit 1 bis 8 Kohlenstoffatomen, ein Cycloalkyl mit 4 bis 10 Kohlenstoff-Atomen, einen aromatischen Rest, wie Phenyl, Benzyl oder Phenethyl darstellen, $R_7$ für $R'_6$, $COR'_6$, $COOR'_6$ oder $CONHR'_6$ steht und $R_8$ Wasserstoff oder einen Rest wie $R''_6$, $COR'_6$, $CO_2R'_6$, $CONHR'_6$, $SO_2R'_6$ oder $SO_2NR'_6R''_6$ darstellt, mit den folgenden Beschränkungen:

- wenn Y $COR_6$ darstellt, wenn X fehlt und wenn $R_3$ Wasserstoff darstellt, dann muss $R_1$ von $CH_2CH_2N(R_4R_5)$ verschieden sein;
- wenn $R''_6$ einen aromatischen Rest darstellt, dann muss $R_1$ von $CH_2CH_2N(R_4R_5)$ verschieden sein und
- Y ist von einer Alkoxygruppe verschieden,

ihre Salze, Hydrate, Solvate, die für die therapeutische Verwendung annehmbar sind,
wobei die Verbindungen der Formel (I), die ein oder mehrere Asymmetriezentren enthalten, isomere Formen annehmen, wobei die Racemate und die reinen Enantiomere dieser Verbindungen ebenfalls Teil dieser Erfindung sind.

**2.** Verbindung der Formel (I) nach Anspruch 1, ausgewählt aus:

Ethyl-4-[3-(2-aminoethyl)-1H-indol-5-yloxymethyl]benzoat;

Methyl-4-[3-(2-aminoethyl)-1H-indol-5-yloxymethyl]benzoat;
{4-[3-(2-Aminoethyl)-1H-indol-5-yloxymethyl]phenyl}methanol;
Propyl-4-[3-(2-aminoethyl)-1H-indol-5-yloxymethyl]benzoat;
2-[5-(4-Nitrobenzyloxy)-1H-indol-3-yl]ethylamin;
2-[5-(4-Methyl-3-nitrobenzyloxy)-1H-indol-3-yl]ethylamin;
Methyl-3-[3-(2-aminoethyl)-1H-indol-5-yloxymethyl]benzoat;
2-[5-(2-Nitrobenzyloxy)-1H-indol-3-yl]ethylamin;
Ethyl-2-[3-(2-aminoethyl)-1 H-indol-5-yloxymethyl]benzoat;
2-[3-(2-Aminoethyl)-1H-indol-5-yloxymethyl]-1-phenylethanon;
2-[3-(2-Aminoethyl)-1H-indol-5-yloxy]-1-(4-methoxyphenyl)ethanon;
Ethyl-4-[3-(2-aminoethyl)-1H-indol-5-yloxy]butyrat;
Ethyl-5-[3-(2-aminoethyl)-1H-indol-5-yloxy]pentanoat;
Ethyl-6-[3-(2-aminoethyl)-1H-indol-5-yloxy]hexanoat;
Benzyl-6-[3-(2-aminoethyl)-1H-indol-5-yloxy]hexanoat;
Isopropyl-6-[3-(2-aminoethyl)-1H-indol-5-yloxy]hexanoat;
6-[3-(2-Aminoethyl)-1H-indol-5-yloxy]hexannitril;
6-[3-(2-Aminoethyl)-1H-indol-5-yloxy]hexylamin;
N-{6-[3-(2-Aminoethyl)-1H-indol-5-yloxy]hexyl}methansulfonamid;
6-[3-(2-Aminoethyl)-1H-indol-5-yloxy]-N-ethylhexanamid;
Isopropyl-5-[3-(2-aminoethyl)-1H-indol-5-yloxy]pentanoat;
Benzyl-5-[3-(2-aminoethyl)-1H-indol-5-yloxy]pentanoat;
4-[3-(2-Aminoethyl)-1H-indol-5-yloxy]butylacetat;
4-[3-(2-Aminoethyl)-1H-indol-5-yloxy]butan-1-ol;
5-[3-(2-Aminoethyl)-1H-indol-5-yloxy]-N-ethylpentanamid;
5-[3-(2-Aminoethyl)-1H-indol-5-yloxy]pentannitril;
5-[3-(2-Aminoethyl)-1H-indol-5-yloxy]pentylamin;
N-{5-[3-(2-Aminoethyl)-1H-indol-5-yloxy]pentyl}methansulfonamid;
Methyl-(±)-[3-(2-aminoethyl)-1H-indol-5-yloxy]phenylacetat;
(±)-2-[3-(2-Aminoethyl)-1H-indol-5-yloxy]-2-phenylethanol;
(±)-[3-(2-Aminoethyl)-1H-indol-5-yloxy]phenylessigsäure;
(±)-2-[3-(2-Aminoethyl)-1H-indol-5-yloxy]-2-phenylacetamid;
Ethyl-(±)-[3-(2-aminoethyl)-1H-indol-5-yloxy]phenylacetat;
Ethyl-(+)-[3-(2-aminoethyl)-1H-indol-5-yloxy]phenylacetat;
Ethyl-(-)-[3-(2-aminoethyl)-1H-indol-5-yloxy]phenylacetat;
Ethyl-5-[3-(1-methylpiperidin-4-yl)-1H-indol-5-yloxy]pentanoat;
Ethyl-5-(3-amino-1,2,3,4-tetrahydrocarbazol-6-yloxy)pentanoat;
Ethyl-(R)-5-[3-(N-methylpyrrolidin-2-ylmethyl)-1H-indol-5-yloxy]pentanoat;

ihre Salze, Hydrate, Solvate, die für die therapeutische Anwendung annehmbar sind.

**3.** Verbindungen nach Anspruch 1, **dadurch gekennzeichnet, dass** $R_1$ für $R_4R_5N\text{-}CH_2\text{-}CH_2\text{-}$ steht.

**4.** Verbindungen nach Anspruch 1, **dadurch gekennzeichnet, dass** $R_4$ und $R_5$ Wasserstoff oder Methyl darstellen.

**5.** Verbindungen nach Anspruch 1, **dadurch gekennzeichnet, dass** $R_2$ Wasserstoff darstellt.

**6.** Verbindungen nach Anspruch 1, **dadurch gekennzeichnet, dass** X eine lineare oder verzweigte Alkylkette darstellt, die 1 bis 8 Kohlenstoff-Atome enthält.

**7.** Verbindungen nach Anspruch 1, **dadurch gekennzeichnet, dass** X ein Phenyl darstellt, an dem der Substituent Y in para-Position angeknüpft ist.

**8.** Verbindungen nach Anspruch 1, **dadurch gekennzeichnet, dass** Y für $COR_6$ oder $OCOR'_6$ steht.

**9.** Verbindungen nach Anspruch 1, **dadurch gekennzeichnet, dass** Y für $NHSO_2R'_6$ steht.

**10.** Verbindungen nach einem der Ansprüche 1 bis 9 im Zustand von Salz, das für die therapeutische Verwendung annehmbar ist, **dadurch gekennzeichnet, dass** diese Salze Hydrochloride, Hydrobromide, Sulfate, Methansulfonate, Fumarate, Maleate oder Succinate sind.

**11.** Verfahren zur Herstellung von Verbindungen der Formel (I) nach Anspruch 1, **dadurch gekennzeichnet, dass** man ein Zwischenprodukt der allgemeinen Formel (II)

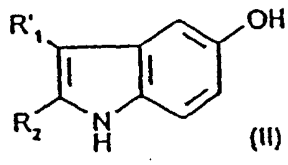

(II)

in der $R_2$ wie vorstehend definiert ist und $R'_1$ gleich $R_1$ oder einer Vorstufe von $R_1$ sein kann (das am Ende der Synthese durch eine geeignete Reaktion, wie beispielsweise das Abspalten einer Schutzgruppe, wiederhergestellt wird) mit einem Derivat der allgemeinen Formel (III)

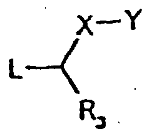

(III)

umsetzt, in der X, Y und $R_3$ wie vorstehend definiert sind und L eine Abgangsgruppe, wie ein Halogen (Iod, Brom oder Chlor), Mesylat, Tosylat oder Sulfat darstellt.

**12.** Verfahren zur Herstellung von Verbindungen der Formel (I) gemäß Anspruch 1, **dadurch gekennzeichnet, dass** man eine Verbindung der allgemeinen Formel (I) oder ein Salz oder ein Derivat einer derartigen Verbindung, das eine Schutzgruppe umfasst, in eine andere Verbindung der allgemeinen Formel (I) gemäß Anspruch 1 überführt.

**13.** Pharmazeutische Zusammensetzungen, die als Wirkstoff eine Verbindung nach einem der Ansprüche 1 bis 10 in Kombination mit einem pharmazeutisch annehmbaren Vehikel enthalten, als Medikamente.

**14.** Pharmazeutische Zusammensetzungen, die als Wirkstoff eine Verbindung nach einem der Ansprüche 1 bis 10 in Kombination mit einem pharmazeutisch annehmbaren Vehikel enthalten, für die sowohl heilende als auch vorbeugende Behandlung von Störungen, die mit Serotonin verbunden sind.

**15.** Pharmazeutische Zusammensetzungen, die als Wirkstoff eine Verbindung nach einem der Ansprüche 1 bis 10 in Kombination mit einem pharmazeutisch annehmbaren Vehikel enthalten, für die sowohl heilende als auch vorbeugende Behandlung von Migräne, von Gefäßschmerz des Gesichtes und von chronischen vaskulären Kopfschmerzen.

**16.** Pharmazeutische Zusammensetzungen, die als Wirkstoff eine Verbindung nach einem der Ansprüche 1 bis 10 in Kombination mit einem pharmazeutisch annehmbaren Vehikel enthalten, für die sowohl heilende als auch vorbeugende Behandlung von Depression, von Zwangsstörungen, von Angst und von Panikattacken.

**17.** Pharmazeutische Zusammensetzungen, die als Wirkstoff eine Verbindung nach einem der Ansprüche 1 bis 10 in Kombination mit einem pharmazeutisch annehmbaren Vehikel enthalten, für die sowohl heilende als auch vorbeugende Behandlung der Schizophrenie, der Spastik, der Aggressivität und/oder des Alkoholismus und/oder von asozialem Verhalten, Ernährungsstörungen, wie Bulimie und Anorexia, sexuellen Dysfunktionen.

**18.** Pharmazeutische Zusammensetzungen, die als aktiven Bestandteil eine Verbindung nach einem der Ansprüche 1 bis 10 in Kombination mit einem pharmazeutisch annehmbaren Vehikel enthalten, für die sowohl heilende als auch vorbeugende Behandlung von neurodegenerativen Krankheiten, wie beispielsweise Parkinson-Krankheit oder Alzheimer-Krankheit.

## Claims

1. Compounds corresponding to the general formula (I)

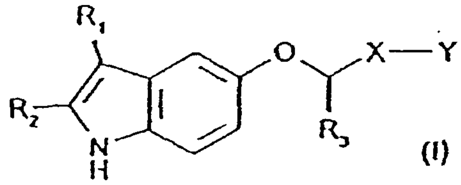

(I)

in which
R1 represents an amino residue corresponding to one of the formulae (i) to (v):

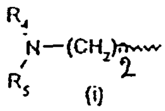

(i)

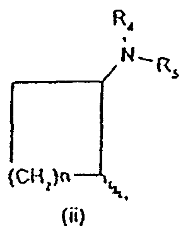

(ii)

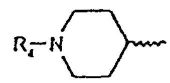

(iii)

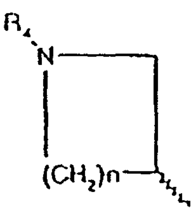

(iv)

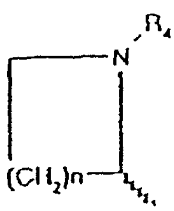

(v)

in which n represents an integer between 1 and 5,
R4 represents a hydrogen, a linear or branched alkyl group comprising from 1 to 6 carbon atoms or a residue of the $(CH_2)_mOR'$ type in which m represents an integer between 1 and 5, and R' a linear or branched alkyl group comprising from 1 to 6 carbon atoms,
R5 represents a hydrogen, or a linear or branched alkyl group comprising from 1 to 6 carbon atoms,
$R_2$ represents a hydrogen, or $R_1$ and $R_2$, taken together, form a ring with 6 carbon atoms substituted with an amine functional group ($NR_4R_5$), provided that when R1 represents an amino residue of formula (I), $R_2$ represents a hydrogen atom.
$R_3$ represents a hydrogen, an alkyl residue comprising from 1 to 5 carbon atoms or a phenyl.
X may be omitted or may represent either a linear or branched alkyl chain comprising from 1 to 8 carbon atoms, or a phenyl which may be variously substituted at various positions with a linear or branched alkyl group comprising from 1 to 6 carbon atoms, a phenyl, a halogen, an alcohol (OH), a nitrile (CN), a nitro ($NO_2$), a thiol (SH),
Y represents a carbonyl-containing ($COR_6$), sulphonyl-containing ($SO_2R_6$), oxygenated ($OR_7$), amino ($NHR_8$), nitrile (CN), nitro ($NO_2$), oxime (C=NOH) or hydroxylamine (NHOH) residue in which $R_6$ represents $R'_6$, $OR'_6$ or $NHR''_6$ where $R'_6$ and $R''_6$ represent a linear or branched alkyl chain from 1 to 8 carbon atoms, a cycloalkyl from 4 to 10 carbon atoms, an aromatic residue such as a phenyl, a benzyl, or a phenethyl, $R_7$ represents $R'_6$, $COR'_6$, $COOR'_6$ or $CONHR'_6$ and $R_8$ represents a hydrogen or a residue such as $R''_6$, $COR'_6$, $CO_2R'_6$, $CONHR'_6$, $SO_2R'_6$ or $SO_2NR'_6R''_6$ with the following restrictions:

> when Y represents $COR_6$, when X is omitted and when $R_3$ represents a hydrogen, then $R_1$ should be different from $CH_2CH_2N(R_4R_5)$,

> when $R''_6$ represents an aromatic residue, then $R_1$ should be different from $CH_2CH_2N(R_4R_5)$ and
> Y is different from an alkoxy group,

their salts, hydrates, solvates which are acceptable for therapeutic use,
the compounds of formula (I) containing 1 or more asymmetric centres have isomeric forms. The racemates and the pure enantiomers of these compounds also form part of this invention.

**2.** Compound of formula (I) according to Claim 1, selected from:

Ethyl 4-[3-(2-aminoethyl)-1H-indol-5-yloxymethyl] benzoate.
Methyl 4-[3-(2-aminoethyl)-1H-indol-5-yloxymethyl] benzoate.
{4-[3-(2-Aminoethyl)-1H-indol-5-yloxymethyl]phenyl} methanol.
Propyl 4-[3-(2-aminoethyl)-1H-indol-5-yloxymethyl] benzoate.
2-[5-(4-Nitrobenzyloxy)-1H-indol-3-yl]ethylamine.
2-[5-(4-Methyl-3-nitrobenzyloxy)-1H-indol-3-yl] ethylamine.
Methyl 3-[3-(2-aminoethyl)-1H-indol-5-yloxymethyl] benzoate.
2-[5-(2-Nitrobenzyloxy)-1H-indol-3-yl]ethylamine.
Ethyl 2-[3-(2-aminoethyl)-1H-indol-5-yloxymethyl] benzoate.
2-[3-(2-Aminoethyl)-1H-indol-5-yloxymethyl]-1-phenylethanone.
2-[3-(2-Aminoethyl)-1H-indol-5-yloxy]-1-(4-methoxyphenyl)ethanone.
Ethyl 4-[3-(2-aminoethyl)-1H-indol-5-yloxy)butyrate.
Ethyl 5-[3-(2-aminoethyl)-1H-indol-5-yloxy]pentanoate.
Ethyl 6-[3-(2-aminoethyl)-1H-indol-5-yloxy]hexanoate.
Benzyl 6-[3-(2-aminoethyl)-1H-indol-5-yloxy]hexanoate.
Isopropyl 6-[3-(2-aminoethyl)-1H-indol-5-yloxy]hexanoate.
6-[3-(2-Aminoethyl)-1H-indol-5-yloxy]hexanitrile.
6-[3-(2-Aminoethyl)-1H-indol-5-yloxy]hexylamine.
N-{6-[3-(2-Aminoethyl)-1H-indol-5-yloxy]hexyl}methanesulphonamide.
6-[3-(2-Aminoethyl)-1H-indol-5-yloxy]-N-ethylhexanamide.
Isopropyl 5-[3-(2-aminoethyl)-1H-indol-5-yloxy] pentanoate.
Benzyl 5-[3-(2-aminoethyl)-1H-indol-5-yloxy]pentanoate.
Acetate of 4-[3-(2-aminoethyl)-1H-indol-5-yloxy]butyl.
4-[3-(2-Aminoethyl)-lH-indol-5-yloxy]butan-1-ol.
5-[3-(2-Aminoethyl)-1H-indol-5-yloxy)-Nethylpentanamide.
5-[3-(2-Aminoethyl)-1H-indol-5-yloxy]pentanenitrile.
5-[3-(2-Aminoethyl)-1H-indol-5-yloxy]pentylamine.
N-{5-[3-(2-Aminoethyl)-1H-indol-5-yloxy]pentyl}methanesulphonamide.
Methyl (±)-[3-(2-aminoethyl)-1H-indol-5-yloxy]phenylacetate.
(±)-2-[3-(2-Aminoethyl)-1H-indol-5-yloxy]-2-phenylethanol.
(±)-[3-(2-Aminoethyl)-1H-indol-5-yloxy]-phenylacetic.
(±)-2-[3-(2-Aminoethyl)-1H-indol-5-yloxy]-2-phenylacetamide.
Ethyl (±)-[3-(2-aminoethyl)-1H-indol-5-yloxy]-phenylacetate.
Ethyl (+)-[3-(2-aminoethyl)-1H-indol-5-yloxy]-phenylacetate.
Ethyl (-)-[3-(2-aminoethyl)-1H-indol-5-yloxy]-phenylacetate.
Ethyl 5-[3-(1-methylpiperidin-4-yl)-1H-indol-5-yloxy]pentanoate.
Ethyl 5-(3-amino-1,2,3,4-tetrahydrocarbazole-6-yloxy)pentanoate.
Ethyl (R)-5-[3-(N-methylpyrrolidin-2-ylmethyl)-1H-indol-5-yloxy]pentanoate,

their salts, hydrates, solvates which are acceptable for therapeutic use.

**3.** Compounds according to Claim 1, **characterized in that** $R_1$ represents

$$R_4R_5N\text{-}CH_2\text{-}CH_2\text{-}$$

**4.** Compounds according to Claim 1, **characterized in that** $R_4$ and $R_5$ represent a hydrogen or a methyl.

**5.** Compounds according to Claim 1, **characterized in that** $R_2$ represents a hydrogen.

6. Compounds according to Claim 1, **characterized in that** X represents a linear or branched alkyl chain comprising from 1 to 8 carbon atoms.

7. Compounds according to Claim 1, **characterized in that** X represents a phenyl onto which the Y substituent is attached at the para position.

8. Compounds according to Claim 1, **characterized in that** Y represents $COR_6$ or $OCOR'_6$.

9. Compounds according to Claim 1, **characterized in that** Y represents $NHSO_2R'_6$.

10. Compounds according to one of Claims 1 to 9, in the form of a salt acceptable for therapeutic use, **characterized in that** these salts are hydrochlorides, hydrobromides, sulphates, methanesulphonates, fumarates, maleates or succinates.

11. Process for the preparation of compounds of formula (I) according to Claim 1, **characterized in that** an intermediate of general formula (II)

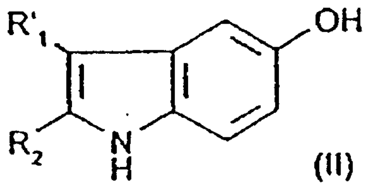

(II)

in which $R_2$ is defined as above and $R'_1$ may be equivalent to $R_1$ or to a precursor of $R_1$ (which will be restored at the end of the synthesis by an appropriate reaction such as for example the cutting of a protective group) is reacted with a derivative of

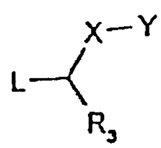

(III)

general formula (III).
in which X, Y and $R_3$ are defined as above and L represent a leaving group such as a halogen (iodine, bromine or chlorine), a mesylate, a tosylate or a sulphate.

12. Process for the preparation of compounds of formula (I) according to Claim 1, **characterized in that** a compound of general formula (I) or a salt or a derivative comprising a group for protecting such a compound, is converted to another compound of general formula (I) according to Claim 1.

13. Pharmaceutical compositions containing, as active ingredients, a compound according to one of Claims 1 to 10, in combination with an acceptable pharmaceutical vehicle as medicaments.

14. Pharmaceutical compositions containing, as active ingredients, a compound according to one of Claims 1 to 10, in combination with an acceptable pharmaceutical vehicle, for both the curative and preventive treatment of disorders related to serotonin.

15. Pharmaceutical compositions containing, as active ingredients, a compound according to one of Claims 1 to 10, in combination with an acceptable pharmaceutical vehicle, for both the curative and preventive treatment of migraine, vascular facial pain and chronic vascular cephalalgia.

16. Pharmaceutical compositions containing, as active ingredients, a compound according to one of Claims 1 to 10,

in combination with an acceptable pharmaceutical vehicle, for both the curative and preventive treatment of depression, obsessive compulsive disorders, anxiety and panic attacks.

**17.** Pharmaceutical compositions containing, as active ingredients, a compound according to one of Claims 1 to 10, in combination with an acceptable pharmaceutical vehicle, for both the curative and preventive treatment of schizophrenia, spasticity, aggressiveness and/or alcoholism and/or antisocial behaviour, dietary disorders such as bulimia and anorexia, sexual dysfunctions.

**18.** Pharmaceutical compositions containing, as active ingredients, a compound according to one of Claims 1 to 10, in combination with an acceptable pharmaceutical vehicle, for both the curative and preventive treatment of neurodegenerative diseases such as for example Parkinson's disease or Alzheimer's disease.